# EUROPÄISCHE PATENTSCHRIFT

(11) **EP 3 003 426 B1**
(45) Veröffentlichungstag und Bekanntmachung des Hinweises auf die Patenterteilung: **10.03.2021**
(21) Anmeldenummer: 13727106.0
(22) Anmeldetag: 30.05.2013
(51) Int. Cl.: A61M 5/148, A61M 5/142

(54) **VORRICHTUNG ZUR ABGABE EINES FLUIDS AN EINEN PATIENTEN**
DEVICE FOR DISPENSING A FLUID TO A PATIENT
DISPOSITIF DE DISTRIBUTION DE FLUIDE À UN PATIENT

(43) Veröffentlichungstag der Anmeldung: 13.04.2016
(73) Patentinhaber: SHL Medical AG, 6300 Zug (CH)
(72) Erfinder: WEIBEL, Ludwig, Daniel, 9104 Waldstatt (CH)
(86) Internationale Anmeldenummer: PCT/EP2013/061153
(87) Internationale Veröffentlichungsnummer: WO 2014/191038

(56) Entgegenhaltungen:
- WO-A2-02/40083
- AU-A1- 2010 207 762
- US-A1- 2011 043 357
- US-B1- 6 659 978

## Beschreibung

Die Erfindung betrifft eine Vorrichtung zur Abgabe eines Fluids an einen Patienten gemäss den Oberbegriffen der unabhängigen Ansprüche.

Bei der Verabreichung von flüssigen Arzneimitteln ist es oft erforderlich, sehr genau dosierte Kleinstmengen des Arzneimittels abzugeben. Häufig müssen die Arzneimittel dabei in den Körper eines Patienten injiziert werden. Dies ist insbesondere bei Arzneimitteln der Fall, welche bei oraler Abgabe ihre Wirksamkeit ganz oder teilweise verlieren. Zu diesen Arzneimitteln zählen insbesondere Proteine (z.B. Insulin, Wachstumshormone, Interferone), Kohlehydrate (z.B. Heparin), Antikörper oder die meisten Impfstoffe. Zur parenteralen Injektion kommen Injektionsspritzen, Arzneimittelstifte (so genannte "Pens") oder Arzneimittelpumpen zum Einsatz.

Während zur einmaligen Verwendung vorgesehene Injektionsspritzen aufgrund des geringen Wartungsaufwandes und der weltweiten Verfügbarkeit gewisse Vorteile aufweisen, verlangen sie dennoch eine gewisse Erfahrung des Benutzers für eine fehlerfreie Applikation. Insbesondere muss das Arzneimittel gegebenenfalls aus entsprechenden Ampullen aufgezogen werden, wobei z.B. auf eine ausreichende Blasenfreiheit und sterile Bedingungen zu achten ist. Dies ist insbesondere für Patienten mit Diabetes heikel, welche sich oft mehrmals täglich unter stark wechselnden Bedingungen eine Insulindosis verabreichen müssen.

Injektionsspritzen werden daher bei regelmässig zu verabreichenden Arzneimitteln mehr und mehr von so genannten Arzneimittelpens verdrängt. Arzneimittelpens haben den Vorteil, dass ein auswechselbarer Arzneimittelbehälter (z.B. Ampulle, Karpulle), umfassend eine ausreichende Arzneimittelmenge für mehrere Dosen, direkt in den Arzneimittelpen eingesetzt wird. Derartige Behälter können z.B. vorgefüllt und steril ausgeliefert werden. Beim Einsetzen des Behälters wird eine Fluidverbindung zwischen dem Arzneimittelpen und dem Behälter hergestellt. Zur Verabreichung wird von einem Injektionsmechanismus jeweils eine vordefinierte Menge des Arzneimittels z.B. mittels einer Kolbenstange aus dem Behälter entnommen und verabreicht. Derartige Arzneimittelpens verlangen beim Wechsel des Arzneimittelbehälters oft eine erhöhte Sorgfalt, da der Injektionsmechanismus in eine Ausgangslage zurückgestellt werden muss. Zudem besteht das Risiko von mechanischen Fehlfunktionen, da derartige Arzneimittelpens häufig täglich eingesetzt und vom Benutzer z.B. in einer Tasche bei sich getragen werden.

Arzneimittelpumpen sind dazu ausgebildet, während längerer Zeit am Körper des Patienten angebracht getragen zu werden (siehe z.B. WO 2008/04078 A1). Derartige Arzneimittelpumpen umfassen typischerweise einen Behälter für das flüssige Arzneimittel sowie eine Pumpe, welche das Arzneimittel zu einem Anschluss der Vorrichtung oder zu einem Injektionssystem fördert. Das Injektionssystem kann dabei eine Verweilkanüle umfassen, welche während des gesamten Verabreichungszeitraums im Körper des Patienten verweilt.

Arzneimittelpumpen haben den Vorteil, dass das Arzneimittel kontinuierlich in Kleinstmengen abgegeben werden kann und unterscheiden sich somit grundsätzlich von Injektionsspritzen oder Arzneimittelpens wie z.B. Insulinpens mit Einzelabgaben zu festgelegten Zeitpunkten. Allerdings ist oft die gewünschte Zuverlässigkeit der Pumpe nicht mit ausreichender Sicherheit gewährleistet oder die Genauigkeit der abgegeben Arzneimittelmenge entspricht nicht den medizinischen Vorgaben. Zudem verlangt das Setzen der Verweilkanüle oft eine gewisse Erfahrung des Benutzers und es besteht das Risiko, dass eine Fluidverbindung zwischen Arzneimittelpumpe und Verweilkanüle unterbrochen wird.

Um das Setzen der Verweilkanüle zu vereinfachen und komfortabel zu gestalten, weisen verschiedene bekannte Arzneimittelpumpen integrierte Injektionsvorrichtungen auf (siehe z.B. WO 2003/090509 A2). Dabei wird die Verweilkanüle bei Inbetriebnahme der Vorrichtung von einem Antrieb automatisch gesetzt. Die weiche Verweilkanüle ist dabei häufig von einer harten Einstechspitze gestützt, welche zum Setzen aus und wieder eingefahren wird. Die harte Einstechspitze dient zur Penetration der Haut des Patienten. Die weiche Verweilkanüle bleibt im Patienten, wenn die Einstechspitze zurückgezogen wird.

Die AU 2010/207762 A1 beschreibt ebenfalls eine Vorrichtung zur Abgabe eines Fluids an einen Patienten. Diese kann an dessen Körperoberfläche angebracht und über eine Fernbedienung gesteuert werden. Die Vorrichtung umfasst Behälter für das Fluid, eine Fördervorrichtung, eine Kontrolleinheit, eine Empfangseinheit, sowie eine Batterie. Die Fördervorrichtung ist über eine proximale Leitung und eine distale Leitung mit einer Verweilkanüle verbunden. In der Verweilkanüle befindet sich eine Einstechkanüle zur Penetration der Haut des Patienten, welche fest an ein Verbindungsstück angebracht ist. Durch Betätigung eines Bedienelementes kann die Einstechkanüle in die Haut des Patienten eingebracht und danach auch wieder zurückgezogen werden.

Während derartige Injektionsvorrichtungen bereits einen recht hohen Benutzungskomfort bieten, weisen gängige Injektionsvorrichtungen oft eine ungleichmässige Einstechbewegung auf, womit sich das Setzen der Verweilkanüle schmerzhaft gestalten kann. Zudem besteht das Risiko einer Beschädigung der Verweilkanüle beim Setzen, insbesondere auch durch die harte Einstechspitze, wenn diese z.B. beim Zurückziehen gegen die Innenwand der Verweilkanüle stösst.

Da derartige Arzneimittelpumpen über längere Zeit am Körper getragen werden, besteht allgemein das Bedürfnis, sowohl Injektionsvorrichtung wie auch die weiteren Elemente der Arzneimittelpumpe, insbesondere die Pumpe selbst, möglichst robust auszugestalten. Aufgrund der oft lebenserhaltenden Funktion der Arzneimittelabgabe bestehen zudem erhöhte Anforderungen an eine Zuverlässigkeit der Vorrichtung im Betrieb. Aufgrund der erforderlichen Miniaturisierung und der üblicherweise zumindest teilweisen Ausführung als Wegwerfeinheiten, besteht das Bedürfnis, eine möglichst einfache Konstruktion bereitzustellen.

Es ist daher eine Aufgabe der Erfindung, die Nachteile des Standes der Technik zu überwinden. Insbesondere ist es eine Aufgabe, eine Vorrichtung zur Abgabe eines Fluids an einen Patienten bereitzustellen, welche sowohl zuverlässig als auch robust und komfortabel in der Handhabung ist. Zudem soll die Vorrichtung eine einfache Konstruktion aufweisen und kostengünstig herstellbar sein.

Diese Aufgaben werden durch eine Vorrichtung mit den Merkmalen der unabhängigen Ansprüche gelöst.

Ein Aspekt der Erfindung betrifft eine Vorrichtung zur Abgabe eines Fluids an einen Patienten umfassend einen, insbesondere zumindest teilweise kollabierbaren, Behälter mit einem Innenraum zur Aufnahme des Fluids. Der Behälter umfasst ein, insbesondere fest im Behälter angeordnetes, Verschlussstück, an welchem eine Abgabeöffnung ausgebildet ist, über welche das Fluid aus dem Innenraum abgebbar ist. Die Vorrichtung umfasst eine von einem Förderantrieb angetriebene Fördervorrichtung zur Förderung des Fluids aus dem Innenraum des Behälters. Die Vorrichtung zeichnet sich dadurch aus, dass die Fördervorrichtung fluidmässig zwischen dem Innenraum und der Abgabeöffnung angeordnet ist, sodass das Fluid mittels der Fördervorrichtung aus dem Innenraum zur Abgabeöffnung förderbar ist.

Eine "fluidmässige Anordnung" bezeichnet hierbei eine Anordnung im Sinne eines fluidkommunizierenden Zusammenwirkens der Komponenten. Eine "fluidmässige Anordnung" eines Elements zwischen zwei Komponenten weist somit wenigstens einen Fluidweg auf, welcher von einer Komponente zur anderen über das Element führt.

Vorliegend ist die Fördervorrichtung fluidmässig zwischen den Innenraum des Behälters und dessen am Verschlussstück ausgebildeter Abgabeöffnung angeordnet. Auf diese Weise kann die Fördervorrichtung ganz oder teilweise am Behälter, insbesondere im Verschlussstück, ausgebildet sein. Der Förderantrieb kann ausserhalb des Behälters angeordnet sein und z.B. über ein Kopplungsmittel an die Fördervorrichtung gekoppelt oder koppelbar sein. Grundsätzlich sind aber auch Lösungen denkbar, bei welchen die Fördervorrichtung an den Behälter koppelbar ist, um die erfindungsgemässe fluidmässige Anordnung zwischen dem Innenraum und der Abgabeöffnung zu erreichen.

Das Verschlussstück kann dazu genutzt werden, um Teile der Fördervorrichtung in das Verschlussstück zu integrieren. Dabei müssen z.B. keine oder nur wenige fluiddichte Verbindungen geschaffen werden, welche anfällig für Leckagen sein können. Bei geeigneter Ausbildung kann die Vorrichtung beispielsweise nur eine fluiddichte Verbindung erfordern, welche z.B. die Abgabeöffnung des Behälters mit einer Injektionsvorrichtung zur z.B. subkutanen Verabreichung des Fluids verbindet.

Allfällig vorhandene Fluidkanäle, welche den Innenraum und/oder die Abgabeöffnung mit der Fördervorrichtung verbinden, können ebenfalls im Verschlussstück ausgebildet sein. Das Verschlussstück kann daher z.B. als einstückiges Spritzgussteil ausgebildet sein, in welchem z.B. Teile der Fördervorrichtung und/oder an diese anschliessende Fluidkanäle und Fluidöffnungen fest miteinander verbunden ausgebildet sein können. Auf diese Weise können fluidführende Komponenten in den Behälter integriert sein, womit z.B. eine Baugrösse der Vorrichtung reduziert und die Zuverlässigkeit erhöht werden kann.

Eine, zumindest teilweise, kollabierbare Ausführung des Behälters hat den Vorteil, dass keine Belüftungsöffnungen vorhanden sein müssen, um zum Ausgleich eines aufgrund der Entnahme des Fluids entstehenden Unterdrucks durch Nachströmen von Luft in den Behälter auszugleichen. Zudem lassen sich kollabierbare Behälter wie z.B. Beutel flach ausbilden, was insbesondere bei tragbaren Vorrichtungen eine Platz sparende Anordnung ergibt.

Der Förderantrieb kann an die Fördervorrichtung koppelbar ausgebildet sein, sodass eine Antriebseinheit von einer Abgabeeinheit der Vorrichtung trennbar ausgebildet sein kann. Dies kann z.B. bei tragbaren Insulinabgabesystemen vorteilhaft sein, bei welchen die Antriebseinheit als wieder verwendbares Modul an jeweils unbenutzte Abgabeeinheiten ankoppelbar sein kann. Die Antriebseinheit umfasst bevorzugt auch einen Energiespeicher für den Förderantrieb sowie eine Steuereinheit für den Förderantrieb bzw. zur Steuerung der Vorrichtung. Ebenso können Kommunikationsmittel in der Antriebseinheit vorgesehen sein, über welche z.B. eine externe Bedienungseinheit an die Steuereinheit angebunden werden kann.

Im Verschlussstück des Behälters kann ein Entnahmekanal ausgebildet sein, welcher mit dem Innenraum kommuniziert und fluidmässig an die Fördervorrichtung anschliesst. Ebenso kann im Verschlussstück ein Abgabekanal ausgebildet sein, welcher mit der Abgabeöffnung kommuniziert und an die Fördervorrichtung anschliesst. Der Anschluss an die Fördervorrichtung ist selbstverständlich derart ausgebildet, dass das Fluid von der Fördervorrichtung über den Entnahmekanal aus dem Innenraum in den Abgabekanal und zur Abgabeöffnung förderbar ist. In Varianten kann die Fördervorrichtung auch direkt an den Innenraum anschliessen, z.B. an einer Entnahmeöffnung im Verschlussstück, und/oder direkt an die Abgabeöffnung grenzen. Bevorzugt ist die Fördervorrichtung derart ausgebildet, dass das Fluid aus dem Innenraum, insbesondere über den Entnahmekanal, ansaugbar ist.

Für eine vereinfachte Konstruktion kann die Fördervorrichtung als ventillose Verdrängerpumpe ausgebildet sein. Eine Ausbildung ohne Ventile ist konstruktiv besonderes einfach umzusetzen, was insbesondere bei einem hohen Miniaturisierungsgrad wie z.B. bei tragbaren Insulinabgabesystemen von Vorteil ist.

Die ventillose Verdrängerpumpe kann z.B. eine Rotationskolbenpumpe umfassen. Bevorzugt ist die ventillose Verdrängerpumpe allerdings als Taumelkolbenpumpe mit einem Kolben und einem Hubraum ausgebildet, wobei der Kolben im Betrieb bei jeder Hubbewegung im Hubraum um eine in der Hubrichtung verlaufende Achse eine Drehbewegung ausführt. Derartige Taumelkolbenpumpen weisen eine besonders einfache Konstruktion auf und können daher leicht und kompakt ausgebildet sein. Auf diese Weise kann die Pumpe zumindest teilweise oder vollständig in den Behälter integriert und z.B. im Verschlussstück ausgebildet sein. Zudem können Taumelkolbenpumpen eine hohe Dosiergenauigkeit aufweisen und in jeder Ausrichtung bezüglich einer Schwerkraftrichtung betrieben werden, was insbesondere bei tragbaren Insulinabgabesystemen von Vorteil ist. Mit der Änderung der Antriebsrichtung kann die Einlass-/Auslassrichtung umgekehrt werden. Zudem sind keine Ventile erforderlich, da Zuführungs- und Abführungsöffnungen zum und aus dem Hubraum vom Kolben selbst aufgrund seiner bei der Hubbewegung ausgeführten Drehbewegung verschliessbar sind. Der Kolben bildet somit in der Regel das einzige bewegliche Teil der Pumpe.

Der Kolben umfasst umfangsseitig eine stirnseitig offene Aussparung, welche die jeweilige Zuführungs- oder Abführungsöffnung mit dem vom Kolben bestimmten Verdrängungsraum im Hubraum in Fluidkommunikation bringt. Die Aussparung kann z.B. als mantelseitige Abflachung am Kolben ausgebildet sein. Bevorzugt ist die Aussparung jedoch als axial ausgerichtete Längsrille auf der Mantelfläche des Kolbens ausgebildet. Auf diese Weise können mehrere, azimutal benachbarte Zuführungsöffnungen und Abgabeöffnungen im Hubraum gezielt bedient werden, indem je nach Drehstellung des Kolbens die Längsrille mit jeweils einer der Zuführungsöffnungen oder einer der Abgabeöffnungen kommuniziert. Eine Breite der Längsrille in Umfangsrichtung ist dabei bevorzugt derart bemessen, dass die Öffnungen von der Längsrille zumindest vollständig überdeckbar sind.

Eine axiale Länge der Aussparung ist in der Regel durch den Hub sowie die axiale Anordnung der Öffnungen vorgegeben. Bevorzugt kann bei vollständig eingeschobenem Kolben wenigstens eine der Öffnungen mit der Aussparung kommunizieren, wenn sich der Kolben in der entsprechenden Drehstellung befindet. Die Aussparung kann zur besseren Abdichtung gegenüber einer Innenwand des Hubraums eine Dichtrippe aufweisen, welche die Aussparung bzw. Längsrille mantelseitig umläuft.

Vorzugsweise ist der Hubraum somit in Abhängigkeit einer Drehstellung des Kolbens, insbesondere gegebenenfalls über den Entnahmekanal, mit dem Innenraum oder, insbesondere gegebenenfalls über den Abgabekanal, mit der Abgabeöffnung in Fluidkommunikation bringbar. Der Entnahmekanal mündet dabei z.B. an der Zuführungsöffnung in den Hubraum, während der Abgabekanal an der Abführungsöffnung in den Hubraum mündet. Selbstverständlich kann die Zuführungsöffnung auch direkt in den Innenraum münden und/oder die Abgabeöffnung mit der Abführungsöffnung zusammenfallen.

Bevorzugt ist wenigstens der Hubraum der Taumelkolbenpumpe im Verschlussstück ausgebildet. Auf diese Weise können z.B. auch an den Hubraum anschliessende Fluidkanäle direkt im Verschlussstück ausgebildet sein. Dabei ragt der Kolben mit Vorteil von ausserhalb des Verschlussstücks in den Hubraum.

Insbesondere kann der Kolben der Fördervorrichtung auf diese Weise ausserhalb des Behälters in der Vorrichtung, insbesondere an einem Gehäuse der Vorrichtung, um die in der Hubrichtung verlaufende Achse drehbar und in der Hubrichtung verschiebbar gelagert sein. Die Fördervorrichtung umfasst in diesem Fall Teile des Behälters, z.B. den Hubraum, sowie Teile ausserhalb des Behälters, z.B. Lagerung des Kolbens oder Teile des Kolbens.

Der Förderantrieb der Fördervorrichtung kann einen Drehantrieb umfassen, insbesondere einen elektrischen Drehantrieb, mit welchem die Drehbewegung des Kolbens erzeugbar ist. Die Hubbewegung kann in diesem Fall z.B. über ein Getriebe oder eine mechanische Führung zur Steuerung erreicht werden, welche die Drehbewegung zumindest teilweise in eine Hubbewegung umsetzt. Ebenso sind selbstverständlich auch separate Antriebe denkbar, welche für die Erzeugung der Hub- und der Drehbewegung zuständig sind. Beispielsweise kann der Förderantrieb einen Linearantrieb für die Hubbewegung und einen Drehantrieb für die Drehbewegung umfassen.Ein separater Hubantrieb kann z.B. nach Art eines magnetischen Linearantriebs magnetisch direkt an den Kolben gekoppelt sein.

Es versteht sich, dass der Drehantrieb und/oder weitere Antriebe sowie z.B. der Kolben selbst von in der Vorrichtung angeordneten Überwachungsvorrichtungen überwacht werden können. Auf diese Weise kann z.B. sichergestellt werden, dass eine aktuelle Kolbenstellung einer erwarteten Stellung entspricht.

Gegebenenfalls kann der Drehantrieb über ein Antriebsgetriebe, insbesondere umfassend ein Schneckenrad, an den Kolben ankoppelbar oder angekoppelt sein. Ein Schneckenrad erlaubt z.B. eine Anordnung einer vom Antrieb kommenden Triebachse unter einem Winkel, insbesondere senkrecht, zur Kolbenachse. Auf diese Weise kann der Antriebsstrang vielseitig und an die Platzverhältnisse angepasst angeordnet werden.

Aufgrund des Antriebsgetriebes kann zudem z.B. ein mit dem Kolben fest verbundenes Zahnrad gegenüber einem weiteren Zahnrad verschiebbar sein, ohne dass ein Eingriff der beiden Räder verloren geht. Dies ist insbesondere bei der vorliegend überlagerten Dreh- und Hubbewegung des Kolbens von Vorteil. Das Antriebsgetriebe kann zudem zusätzlich Steuerscheiben oder andere Elemente umfassen, welche z.B. eine Bewegung des Kolbens steuern.

Bevorzugt ist die Hubbewegung des Kolbens mit der Drehbewegung des Kolbens gekoppelt. Die Hubbewegung ist dabei mit Vorteil mit der Drehbewegung zwangsgekoppelt, wobei die Kopplung mechanisch erfolgen kann. In diesem Fall erfolgt die Kopplung mit Vorteil über eine Steuerfläche, welche insbesondere am Verschlussstück des Behälters ausgebildet ist. Ein fest mit dem Kolben verbundener Läufer kann dabei auf der Steuerfläche gleiten. Selbstverständlich sind auch andere bekannte Arten von mechanischen Kopplungen denkbar. Aufgrund der Zwangskopplung kann auf einfache Weise sichergestellt werden, dass sich der Kolben in jeder Hubposition auch in einer gewünschten Drehposition oder umgekehrt befindet.

Die Kopplung kann selbstverständlich, insbesondere im Falle getrennter Antriebe für Hub- und Drehbewegung, auch elektronisch über eine Antriebssteuerung erfolgen. Selbstverständlich können die Hub- und Drehbewegung erforderlichenfalls auch unabhängig voneinander gesteuert sein.

Das Verschlussstück des Behälters kann einen mit dem Innenraum kommunizierenden Füllkanal aufweisen, welcher an einer Füllöffnung des Verschlussstücks verschliessbar nach aussen offen ist. Auf diese Weise kann der leere Behälter z.B. in die Vorrichtung eingesetzt werden, und erst vor Anwendung der Vorrichtung durch einen Benutzer befüllt werden. Es versteht sich, dass auch vorgefüllt Behälter zum Einsatz kommen können, welche im Lieferzustand bereits mit einem Fluid gefüllt in der Vorrichtung angeordnet sind.

Die Füllöffnung kann mit einem Kopplungsmittel zum Ankoppeln einer Fluidquelle, insbesondere einer Luer-Kupplung, versehen sein. Auf diese Weise kann die Fluidquelle wie z.B. eine Spritze oder ein Reservoir, auf einfache Weise an den Behälter angeschlossen werden. Ein Gehäuse der Vorrichtung sowie die Füllöffnung können hierzu entsprechend ausgebildet sein, sodass die Füllöffnung von aussen zugänglich ist. Selbstverständlich kann die Füllöffnung auch ein medizinisches selbst dichtendes Septum umfassen, durch welches das Fluid mit einer Injektionsnadel in den Behälter eingebracht werden kann.

Um einen Rückfluss des Fluids aus dem Behälter zu verhindern, kann eine Ventilvorrichtung im Füllkanal angeordnet sein. Diese kann ein Schnabelventil umfassen, welches den Füllkanal gegen einen Fluidstrom von dem Innenraum nach aussen sperrt. Da der Behälter in der Regel nach dem Befüllen unter Überdrück steht, schliesst das Schnabelventil für die meisten Anwendungen mit ausreichender Dichtigkeit. Zusätzlich kann die Füllöffnung zur Sicherheit mit einem Stopfen verschliessbar sein, um sicherzustellen, dass kein Fluid austreten kann. Selbstverständlich sind auch andere Ventilvorrichtungen denkbar. Beispielsweise kann auch eine Ventilvorrichtung mit zwei Öffnungen zur Anwendung kommen, welche von einer abhebbaren Dichtmembran verschlossen sind (siehe z.B. eine Ventilvorrichtung an einem Verschlussstück eines flexiblen Behälters gemäss der WO 2012/175465 A1).

Selbstverständlich kann die Füllöffnung auch auf andere Weise verschlossen sein. Beispielsweise kann eine Beutelfolie eines teilweise kollabierbar ausgebildeten Behälters dazu benutzt werden, einen unter der Folie angeordneten Füllkanal von ausserhalb des Behälters her abzuklemmen. Hierzu kann z.B. in der Vorrichtung, z.B. innenseitig an einem Gehäuse, ein entsprechender Vorsprung ausgebildet sein, welcher bei geschlossenem Gehäuse auf die Beutelfolie drückt und den Füllkanal abklemmt.

In der Regel reicht es aus, wenn der Behälter einen Innenraum für wenigstens ein Fluid aufweist. Selbstverständlich kann der Behälter aber auch als Mehrkammerbehälter ausgebildet sein. In diesem Fall umfasst der Behälter wenigstens einen weiteren Innenraum, welcher gegenüber dem ersten Innenraum derart abgetrennt ist, dass wenigstens ein weiteres Fluid getrennt vom ersten Fluid im Behälter aufnehmbar ist. Dies kann z.B. bei Medikamenten erforderlich sein, welche erst kurz vor der Verabreichung vermischt werden dürfen. Ebenso ist es grundsätzlich denkbar, dass einer der Innenräume ein Medikament in fester Form, z.B. als Pulver umfasst, während der andere Innenraum ein Lösungsmittel umfasst.

Bevorzugt ist die Fördervorrichtung derart fluidmässig zwischen dem wenigstens einen weiteren Innenraum und entweder der Abgabeöffnung oder wenigstens einer weiteren Abgabeöffnung oder einem anderen der Innenräume angeordnet, sodass das wenigstens eine weitere Fluid mittels der Fördervorrichtung aus dem Innenraum zur Abgabeöffnung oder zur wenigstens einen weiteren Abgabeöffnung oder in den anderen der Innenräume förderbar ist. Dieselbe Fördervorrichtung kann somit zur Förderung des Fluids zwischen den Innenräumen oder zwischen den Innenräumen und einer oder mehrerer Abgabeöffnungen ausgebildet sein. Dies ist insbesondere im Fall einer Taumelkolbenpumpe besonders einfach realisierbar, da diese mit nur einem Kolben und einem Hubraum wie oben beschrieben zur Beschickung mehrerer anschliessender Kanäle ausgebildet sein kann.

Im Falle mehrerer Abgabeöffnungen am Verschlussstück kann z.B. eine Mischung der Fluide erst ausserhalb des Behälters erfolgen. Ebenso kann z.B. an jede Abgabeöffnung ein separates Injektionssystem anschliessen, welches z.B. eine getrennte subkutane Abgabe der Fluide aus jedem Innenraum ermöglicht. Wird das Fluid zur selben Abgabeöffnung gefördert, kann eine Mischung je nach Bedarf z.B. im Behälter erfolgen oder die Fluide können sequenziell über die Abgabeöffnung abgegeben werden. Hierzu kann z.B. an der Abgabeöffnung oder im Abgabekanal ein statischer Mixer vorgesehen sein.

Alternativ, oder bei mehr als zwei Innenräumen zusätzlich, kann wenigstens eine weitere, insbesondere der ersten Fördervorrichtung weitgehend gleichartige, Fördervorrichtung in der Vorrichtung vorhanden sein, welche dem wenigstens einen weiteren Innenraum zugeordnet ist. Die wenigstens eine weitere Fördervorrichtung ist in diesem Fall derart fluidmässig zwischen dem wenigstens einen weiteren Innenraum und der Abgabeöffnung oder wenigstens einer weiteren Abgabeöffnung oder einem anderen der Innenräume angeordnet, dass das wenigstens eine weitere Fluid mittels der wenigstens einen weiteren Fördervorrichtung aus dem wenigstens einen weiteren Innenraum zur Abgabeöffnung respektive zur wenigstens einen weiteren Abgabeöffnung oder in den anderen der Innenräume förderbar ist. Die wenigstens eine weitere Fördervorrichtung kann somit zur Förderung zu einer oder mehreren Abgabeöffnung vorgesehen sein oder aber auch ausschliesslich zur Förderung eines Fluids zwischen zwei der Innenräume. Es erschliessen sich unmittelbar die verschiedenen Kombinationsmöglichkeiten, welche sich aufgrund der zusätzlichen Fördervorrichtung ergeben. Aufgrund der Integrierbarkeit der Fördervorrichtung in den Behälter, insbesondere in das Verschlussstück, können die entsprechend erforderlichen Fluidkanäle sämtlich im Verschlussstück bzw. im Behälter selbst ausgebildet sein(analog dem Fall des Behälters mit einem Innenraum).

Selbstverständlich können auch mehrere Behälter vorhanden sein, wobei jedem Behälter wenigstens eine Fördervorrichtung zugeordnet ist. Die Fördervorrichtungen können dabei gleichartig ausgebildet sein. Indem jedem Behälter wenigstens eine Fördervorrichtung zugeordnet ist, können diese jedoch auch unterschiedlich und an die jeweiligen Erfordernisse wie z.B. an das im zugeordneten Behälter enthaltene Fluid angepasst ausgebildet sein. Die mehreren Behälter können dabei in der Vorrichtung gestapelt oder nebeneinander angeordnet sein.

Im Falle mehrerer Fördervorrichtungen sind die Drehbewegungen der Kolben der Fördervorrichtung, bevorzugt mechanisch, gekoppelt. Auf diese Weise können beide Fördervorrichtungen auf einfache Weise mit nur einem Förderantrieb angetrieben werden. Die Fördervorrichtungen können synchron betreibbar sein oder z.B. über das entsprechend ausgebildete Antriebsgetriebe in einem unterschiedlichen Verhältnis. Es versteht sich, dass auch die Hubbewegungen gekoppelt sein können bzw. dass auch separate Antriebe für Hub- und Drehbewegung für jede der Fördervorrichtungen vorhanden sein können.

Die Vorrichtung kann eine Injektionsvorrichtung zur kontinuierlichen subkutanen Abgabe des Fluids oder gegebenenfalls der Fluide an den Patienten umfassen. Dies ist insbesondere bei einer Ausbildung als z.B. tragbares Abgabesystem zur subkutanen Verabreichung eines flüssigen Medikaments wie z.B. Insulin vorteilhaft. Die Injektionsvorrichtung kann wenigstens eine Injektionskanüle umfassen, welche über einen Infusionsschlauch direkt oder indirekt an die Abgabeöffnung des Behälters anschliessbar ist. Ebenso kann die Vorrichtung auch wenigstens einen Abgabeport umfassen, welcher mit der Abgabeöffnung kommuniziert und an welchen von ausserhalb eines Gehäuses der Vorrichtung her das Injektionssystem angeschlossen werden kann.

Alternativ kann die Vorrichtung auch als stationäre Fluidquelle ohne Injektionssystem ausgebildet sein, welche über einen Abgabeport ein Fluid z.B. mit präziser Dosierung zur Verfügung stellt. Bevorzugt ist die Vorrichtung jedoch als tragbares Abgabesystem zur subkutanen Verabreichung eines flüssigen Medikaments ausgebildet und umfasst eine Injektionsvorrichtung, welche in die Vorrichtung integriert und in einem Lieferzustand innerhalb eines Gehäuses der Vorrichtung angeordnet ist.

Ein weiterer Aspekt der Erfindung betrifft eine Vorrichtung zur Abgabe eines Fluids an einen Patienten, umfassend einen Behälter zur Aufnahme des Fluids, mit einem Gehäuse, welches aussenseitig eine Kontaktfläche aufweist, mit welcher die Vorrichtung an einen Körper des Patienten anlegbar ist. Die Vorrichtung kann dabei wie vorgängig beschrieben ausgebildet sein. Die Vorrichtung umfasst eine Injektionsvorrichtung mit einer von einem distalen Endbereich einer Einstechkanüle gestützten flexiblen transkutanen Verweilkanüle, wobei die Einstechkanüle in einer Bereitschaftsposition im Wesentlichen innerhalb des Gehäuses angeordnet ist und zum Setzen der Verweilkanüle mit dem distalen Endbereich durch eine Setzöffnung an der Kontaktfläche aus dem Gehäuse in eine Setzposition ausfahrbar und wieder in eine Endposition einfahrbar ist. Ein proximaler Endbereich der Einstechkanüle ist fluiddicht an eine Abgabeöffnung angeschlossen, an welcher das Fluid aus dem Behälter abgebbar ist. Dabei ist eine Führungsvorrichtung vorhanden, an welcher der distale Endbereich der Einstechkanüle beim, insbesondere berührungsfreien, Einfahren und Ausfahren durch die Setzöffnung, vorzugsweise verschiebbar, geführt ist.

"Proximal" und "distal" beziehen sich jeweils auf eine aus der Sicht des Behälters bezüglich des Fluidflusses bei der Abgabe nähere (proximale) und fernere (distale) Anordnung.

Die Kontaktfläche der Vorrichtung weist bevorzugt eine Klebefläche auf, welche das Anbringen der Vorrichtung auf der Haut des Patienten ermöglicht. Das Gehäuse kann hierzu eine Flexibilität aufweisen, um das Tragen der Vorrichtung angenehmer zu gestalten. Ebenso kann die Kontaktfläche eine Krümmung aufweisen, um ein komfortableres Anliegen zu gewährleisten.

Die Einstechkanüle ist bevorzugt ausreichend steif ausgebildet, um die Haut des Patienten penetrieren zu können. Hierzu weist die Einstechkanüle an ihrem distalen Ende eine Einstechspitze auf. Im distalen Endbereich ist die Einstechkanüle von der Verweilkanüle ummantelt. Die Verweilkanüle ist flexibel ausgebildet und besteht aus einem bioverträglichen Material ausreichender Festigkeit, um für längere Zeit als transkutane Kanüle im Patienten zu verbleiben. In der Bereitschaftsposition steht die Einstechspitze der Einstechkanüle über die Verweilkanüle vor, sodass beim Setzen der Verweilkanüle die Einstechspitze die Haut des Patienten beim Ausfahren penetrieren kann.

Die Einstechkanüle ist mit ihrem proximalen Endbereich fluiddicht an eine Abgabeöffnung angeschlossen, an welcher das Fluid aus dem Behälter abgebbar ist. In der Endposition ragt das distale Ende der Einstechkanüle in die gesetzte Verweilkanüle, sodass das Fluid aus dem Behälter über die Einstechkanüle der Verweilkanüle zuführbar ist.

Der distale Endbereich der Einstechkanüle tritt beim Setzen der Kanüle beim Ausfahren zusammen mit der daran angeordneten Verweilkanüle durch die Setzöffnung aus der Vorrichtung aus. Dabei kann die Verweilkanüle aufgrund der Führungsvorrichtung weitgehend berührungsfrei durch die Setzöffnung austreten, sodass es zu keinen Umstülpungen bzw. Beschädigungen der Verweilkanüle an der Setzöffnung kommen kann. Beim Einfahren der Einstechkanüle verbleibt die Verweilkanüle in der ausgefahrenen Position. Die Führungsvorrichtung stellt dabei sicher, dass der distale Endbereich entgegen der Einstechrichtung, d.h. entsprechend der Lage der gesetzten Verweilkanüle, einfahrbar ist, sodass die Einstechspitze die Verweilkanüle nicht beschädigen kann.

Es hat sich gezeigt, dass das Setzen der Verweilkanüle besonders komfortabel ist, wenn der distale Endbereich derart ausgebildet und bewegbar ist, dass während des Ausfahrens und des Einfahrens ein Austrittspunkt bezüglich der Kontaktfläche ortsfest und eine Austrittsrichtung am Austrittspunkt bezüglich der Kontaktfläche konstant ist. Auf diese Weise ist sichergestellt, dass der distale Endbereich die Haut des Patienten ohne laterale Verschiebung in einer reinen Stichbewegung penetriert. Der distale Endbereich kann dabei gekrümmt ausgebildet sein, wobei die Einstechbewegung der Krümmung folgt. Ebenso kann der distale Endbereich geradlinig ausgebildet sein, in welchem Fall die Einstechrichtung geradlinig ist. Bevorzugt erfolgt die Einstechbewegung wenigstens an der Kontaktfläche senkrecht zur Kontaktfläche. Es erschliesst sich unmittelbar, dass aufgrund der erfindungemässen Führungsvorrichtung der distale Endbereich der Einstechkanüle unabhängig von einer Ausbildung der Setzöffnung gezielt in der gewünschten Einstechbewegung geführt sein kann.

Die Führungsvorrichtung ist bevorzugt im Innenraum des Gehäuses ausgebildet. Auf diese Weise kann die Führung nicht durch eine fehlerhafte Manipulation durch einen Benutzer gefährdet werden.

Die Führungsvorrichtung umfasst bevorzugt einen, insbesondere im distalen Endbereich, starr mit der Einstechkanüle verbundenen Läufer, welcher in einer Führungsschiene der Führungsvorrichtung verschiebbar geführt ist. Die Führungsschiene kann z.B. als Kurvenführungen ausgebildet sein, an welcher der Läufer auf einer Kurvenbahn geführt verschiebbar ist. Je nach Ausbildung des distalen Endbereichs kann die Führungsschiene aber auch als Linearführung ausgebildet sein.

Bevorzugt ist der distale Endbereich geradlinig ausgebildet, wobei die Führungsvorrichtung als Linearführung ausgebildet ist. Eine geradlinige Einstechbewegung ist mit der Führungsvorrichtung besonders einfach und exakt umzusetzen. Ebenso ist eine geradlinige Ausbildung des distalen Endbereichs besonders einfach herzustellen. Bevorzugt ist die Führungsrichtung der Linearführung senkrecht zur Kontaktfläche, und damit auch zur Hautoberfläche des Patienten, ausgerichtet, sodass sich gesamthaft eine senkrechte Einstechbewegung ergibt.

Mit Vorteil ist der proximale Endbereich der Einstechkanüle weitgehend parallel zum distalen Endbereich ausgerichtet. Bevorzugt weisen dabei die Enden der Einstechkanüle in dieselbe Richtung. Auf diese Weise kann eine optimale Platzausnützung für eine möglichst kompakte Bauweise der Vorrichtung erreicht werden.

Die Einstechkanüle kann als durchgehende Hohlnadel ausgebildet sein. Vorzugsweise ist die Einstechkanüle dabei aus einem Stahl gefertigt. Dies hat den Vorteil, dass eine robuste Fluidverbindung zwischen Abgabeöffnung und der Verweilkanüle bereitgestellt werden kann, ohne dass zusätzlich fluiddichte Verbindungen z.B. mit einem flexiblen Schlauch vorhanden sein müssen.

Bevorzugt ist der proximale Endbereich der Einstechkanüle ortsfest und starr in der Vorrichtung angeordnet. Dies hat den Vorteil, dass die Einstechkanüle direkt an die ortsfest in der Vorrichtung angeordnete Abgabeöffnung anschliessen bzw. angeschlossen sein kann. Damit sind keine unnötigen Fluidverbindungen zwischen Einstechkanüle und Abgabeöffnung erforderlich, welche zusätzliche Dichtungen erfordern und hinsichtlich Leckagen heikel sind.

Die Einstechkanüle weist bevorzugt zumindest zwischen dem proximalen und dem distalen Endbereich eine ausreichende Flexibilität auf, sodass die zum Setzen erforderliche Beweglichkeit des distalen Endbereichs, z.B. aufgrund einer Durchbiegung der Einstechkanüle, bereitgestellt sein kann. Die erfindungsgemässe Führungsvorrichtung stellt dabei sicher, dass die Durchbiegung der Einstechkanüle keinen Einfluss auf die Einstechrichtung des distalen Endbereichs hat.

Je nach Einstechrichtung, insbesondere bei einer geradlinigen Einstechbewegung, kann ein Längenausgleich zwischen dem ortsfest angeordneten proximalen Endbereich und dem distalen Endbereich erforderlich sein. Um auch bei einer durchgehenden Einstechkanüle einen ausreichenden Längenausgleich bereitzustellen, kann die Einstechkanüle wenigstens in der Bereitschaftsposition in einem Mittelbereich zwischen dem proximalen und dem distalen Endbereich gekrümmt ausgebildet sein. Aufgrund des gekrümmten Mittelbereichs kann bereits aufgrund einer, z.B. im Vergleich zur Verweilkanüle, geringen Flexibilität der Einstechkanüle wie es z.B. im Falle von Stahl der Fall sein kann, durch Streckung oder Stauchung des gekrümmten Mittelbereichs ein ausreichender Längenausgleich erreicht werden. Bevorzugt weist die Einstechkanüle im Mittelbereich einen gleich bleibenden Krümmungssinn auf. Selbstverständlich kann in anderen Ausführungsformen der Krümmungssinn z.B. auch alternieren, sodass der Mittelbereich z.B. einer Wellenlinie folgt. Eine Krümmung mit gleich bleibendem Krümmungssinn ist in der Regel jedoch einfacher herzustellen.

Bevorzugt ist ein eingeschlossener Winkel zwischen dem proximalen Endbereich und einem benachbarten Abschnitt des Mittelbereichs sowie ein eingeschlossener Winkel zwischen dem distalen Endbereich und einem benachbarten Abschnitt des Mittelbereichs jeweils kleiner als 90 Grad. Auf diese Weise ist sichergestellt, dass sowohl der proximale als auch der distale Endbereich gegenüber dem Mittelbereich ausreichend abgewinkelt sind, um die zum Setzen erforderliche Beweglichkeit des distalen Endbereichs bei ortsfestem proximalem Endbereich bereitzustellen.

Die Verweilkanüle kann gesamthaft verschiebbar bezüglich der Einstechkanüle im distalen Endbereich angeordnet sein, wobei die Verweilkanüle wenigstens in einem proximalen Endbereich der Verweilkanüle die Einstechkanüle mit einer Gleitdichtung fluiddicht umschliesst. Auf diese Weise ist sichergestellt, dass die Verweilkanüle beim Einfahren der Einstechkanüle aus der Setzposition in die Endposition vom distalen Endbereich zumindest teilweise abziehbar ist. Bei gesetzter Verweilkanüle und eingefahrener Einstechkanüle ist eine fluiddichte Verbindung zwischen der Einstechkanüle und der Verweilkanüle aufgrund der Gleitdichtung sichergestellt. Alternativ kann die Verweilkanüle mit ihrem proximalen Endbereich fest an der Einstechkanüle befestigt sein. In diesem Fall kann die Verweilkanüle einen dehnbaren oder deformierbaren Bereich wie z.B. einen Balg umfassen, welcher bei gesetzter Verweilkanüle das Einfahren des distalen Endbereichs der Einstechkanüle ermöglicht.

Bevorzugt ist die Verweilkanüle, insbesondere selbsttätig, am Gehäuse arretierbar, wenn die Einstechkanüle in der Setzposition ist. Auf diese Weise ist die Verweilkanüle beim Ausfahren automatisch in der Setzposition, insbesondere am Gehäuse, festlegbar. Bevorzugt kann hierzu ein einfacher Schnappmechanismus am Gehäuse, insbesondere bei der Setzöffnung, vorgesehen sein, in welchem die Verweilkanüle verrasten kann. Die Verweilkanüle kann hierzu an ihrem proximalen Ende einen nach aussen ragenden Rastkragen aufweisen.

Beim Einfahren der Einstechkanüle aus der Setzposition in die Endposition verbleibt die arretierte Verweilkanüle in der ausgefahrenen, d.h. in der gesetzten, Position und wird zumindest teilweise von der Einstechkanüle abgezogen. In der Endposition ragt die Einstechkanüle mit ihrem distalen Ende in die Verweilkanüle und bildet aufgrund der Gleitdichtung der Verweilkanüle eine fluiddichte Verbindung mit der Verweilkanüle.

Im proximalen Endbereich der Einstechkanüle kann ein Kopplungsmittel ausgebildet sein, mit welchem die Einstechkanüle an die Abgabeöffnung, insbesondere gegebenenfalls an die Abgabeöffnung des Behälters, angekoppelt ist. Beispielsweise kann ein konischer Kopplungsstutzen an der Einstechkanüle ausgebildet sein, welcher in einen entsprechenden Sitz an der Abgabeöffnung einsetzbar bzw. eingesetzt ist. Alternativ kann die Einstechkanüle z.B. auch in die Abgabeöffnung eingeklebt oder vom Verschlussstück des Behälters umspritzt sein.

Mit Vorteil ist ein Setzantrieb vorhanden, mit welchem der distale Endbereich der Einstechkanüle über ein Setzgetriebe zum Setzen der Verweilkanüle aus- und wieder einfahrbar ist. Der Setzantrieb sowie das Setzgetriebe sind dabei selbstverständlich derart ausgebildet, dass die durch die Führungsvorrichtung vorgegebene Einstechbewegung durch den Setzantrieb ausführbar ist. Selbstverständlich sind auch Ausführungsformen denkbar, bei welchen der Setzantrieb direkt mit der Einstechkanüle, d.h. ohne Setzgetriebe, zusammenwirkt.

Der Setzantrieb kann hierzu einen Drehantrieb und das Setzgetriebe ein Schneckengetriebe umfassen. Der Drehantrieb ist über das Schneckengetriebe an die Einstechkanüle, insbesondere gegebenenfalls an den Läufer der Führungsvorrichtung, ankoppelbar oder angekoppelt. Ein Drehantrieb kann besonders einfach z.B. von einem handelsüblichen Elektromotor bereitgestellt werden. Das Schneckengetriebe hat den Vorteil, dass ein Zahnrad, welches vom Schneckenrad angetrieben ist, auch vom Schneckenrad gesperrt ist. Auf diese Weise kann durch das Setzgetriebe sichergestellt werden, dass die Einstechkanüle in einer einmal eingenommenen Position, insbesondere der Endposition nach dem Setzen der Verweilkanüle, selbst arretierend festgestellt ist.

Der Setzantrieb kann auch einen Linearantrieb und das Setzgetriebe einen schwenkbar gelagerten Kniehebel umfassen, über welchen der Linearantrieb an die Einstechkanüle, insbesondere gegebenenfalls an den Läufer der Führungsvorrichtung, ankoppelbar oder angekoppelt ist. Ein Linearantrieb kann besonders Platz sparend ausgebildet und in der Vorrichtung angeordnet sein. Der Kniehebel bietet den Vorteil einer besonders einfachen Umlenkung einer Bewegung in einer ersten Richtung in eine Bewegung in eine zweite, von der ersten verschiedene Richtung.

Alternativ kann ein Linearantrieb z.B. durch einen einfachen Elektromagneten bereitgestellt werden, welcher ohne Kniehebel direkt auf den Läufer der Führungsvorrichtung wirkt und diesen bei Aktivierung zum Ausfahren der Einstechkanüle anzieht und bei Deaktivierung wieder freigibt. Dabei kann eine Rückstellfeder oder ein zweiter Linearantrieb vorhanden sein, um die Einstechkanüle in die Endposition einzufahren. Ebenso kann das Setzgetriebe z.B. auch eine in Wirkrichtung des Linearantriebs bewegliche Zahnstange umfassen, welche in ein mit der Einstechkanüle zusammenwirkendes Zahnrad eingreift.

Das Setzgetriebe kann eine, insbesondere gegebenenfalls am Läufer oder am Kniehebel, ausgebildete Längsführung aufweisen, welche weitgehend quer zur Einstechrichtung angeordnet ist und in welche ein Nocken des Setzgetriebes in der Längsführung verschiebbar eingreift. Mit dieser Anordnung kann besonders einfach eine Rotationsbewegung in eine Linearbewegung umgesetzt werden. Das Setzgetriebe kann z.B. eine Rotationsscheibe mit einem exzentrisch angeordneten Nocken aufweisen, welcher in die, insbesondere gegebenenfalls am Läufer ausgebildete, Längsführung eingreift. Die Rotationsscheibe kann z.B. über einen umfangsseitig ausgebildeten Zahnkranz oder ein starr und koaxial mit der Rotationsscheibe verbundenes Zahnrad von einem Drehantrieb, z.B. über ein Schneckenrad, oder von einem Linearantrieb, z.B. über eine Zahnstange, angetrieben sein. Bei einer Drehbewegung der Rotationsscheibe kann die Einstechkanüle über den Nocken in der Führungsvorrichtung verschoben werden, wobei gleichzeitig der Nocken in der Längsführung verschoben wird. Da der Nocken während eines Umlaufs der Rotationsscheibe seine Bewegungsrichtung in Einstechrichtung umkehrt, kann die Einfahrbewegung und die Ausfahrbewegung bei gleicher Drehrichtung der Rotationsscheibe ausgeführt werden.

Ebenso kann z.B. gegebenenfalls der Kniehebel die genannte Längsführung aufweisen, in welcher ein fest mit der Einstechkanüle verbundenen Nocken verschiebbar geführt ist. Wird der Kniehebel z.B. von einem Linearantrieb um seine Drehachse geschwenkt, verschiebt der Nocken z.B. die Einstechkanüle in der Führungsvorrichtung in Einstechrichtung, wobei die Längsführung die erforderliche Beweglichkeit des Nockens bereitstellt.

Selbstverständlich können in allen Fällen Überwachungsvorrichtungen vorhanden sein, mit welchen bewegliche Komponenten wie z.B. der Setzantrieb und/oder die Führungsvorrichtung überwachbar sind. Auf diese Weise kann sichergestellt werden, dass eine Antriebsstellung bzw. eine Stellung der Führungsvorrichtung und/oder der Einstechkanüle einer erwarteten Position entspricht.

In jeder Ausführungsform umfasst die Vorrichtung bevorzugt ein Antriebsmodul und ein Abgabemodul, welche durch einen Benutzer verbindbar und voneinander trennbar ausgebildet sind. Dabei umfasst das Antriebsmodul zumindest Teile des Förderantriebs, insbesondere gegebenenfalls den Drehantrieb und/oder gegebenenfalls den Setzantrieb der Injektionsvorrichtung. Das Abgabemodul weist zumindest den Behälter sowie die Fördervorrichtung und gegebenenfalls die Injektionsvorrichtung auf. Bevorzugt umfasst das Antriebsmodul auch eine Batterie zur Versorgung der Antriebe sowie eine Steuerungseinheit zur Steuerung der Vorrichtung, insbesondere der Antriebe. Ebenso können Kommunikationsmittel im Antriebsmodul vorgesehen sein, über welche eine externe Bedienungseinheit an die Steuereinheit anbindbar ist.

Das Antriebsmodul und das Abgabemodul sind bevorzugt derart ausgebildet, dass die Antriebe über entsprechende Kopplungsmittel auf einfache Weise direkt oder indirekt an den Kolben und/oder das Injektionssystem bzw. das Antriebsgetriebe oder das Setzgetriebe ankoppelbar sind. Hierzu können z.B. form- und oder kraftschlüssige Steckkupplungen zum Einsatz kommen. Das Antriebsmodul, welches hygienisch unbedenkliche Komponenten umfasst, kann z.B. wieder verwendbar ausgebildet sein. Das Abgabemodul, welches z.B. das hygienisch relevante Injektionssystem und das Fluid umfasst, kann hingegen als Wegwerfmodul ausgebildet sein. Die beiden Module können zum einfachen Austausch z.B. über eine Schnappkupplung aneinander ankoppelbar und wieder trennbar sein.

Die Erfindung wird im Folgenden anhand von Abbildungen exemplarischer Ausführungsbeispiele näher erläutert. Es zeigen schematisch:
- Fig. 1:: eine erfindungsgemässe Vorrichtung in einer Schrägansicht auf ein Gehäuse von oben;
- Fig. 2:: die Vorrichtung gemäss Fig. 1 in einer Schrägansicht auf eine Kontaktfläche des Gehäuses von unten;
- Fig. 3:: zeigt eine Schrägansicht der Vorrichtung der Fig. 1 ohne Gehäuseschalen;
- Fig. 4:: ein Antriebsmodul der Vorrichtung der Fig. 3 ohne oberen Gehäuseteil.
- Fig. 5:: eine Draufsicht auf die Vorrichtung der Fig. 3 von oben in Richtung senkrecht auf eine Grundplatte 3;
- Fig. 6:: eine Seitenansicht der Vorrichtung der Fig. 3;
- Fig. 7:: eine Frontalansicht auf eine Stirnseite der Vorrichtung der Fig. 3 mit einem Füllport.
- Fig. 8:: eine Ausschnittsvergrösserung eines Abgabebereichs gemäss Fig. 3;
- Fig. 9:: eine Ausschnittsvergrösserung eines Injektionsbereichs gemäss Fig. 3;
- Fig. 10:: eine Schnittansicht in einer Schnittebene gemäss Fig. 5
- Fig. 11:: eine Ausschnittsansicht der Schnittansicht der Fig. 10 in einem Einstechbereich;
- Fig. 12:: eine Seitenansicht der Vorrichtung gemäss Darstellung der Fig. 6 mit ausgeblendetem Setzantrieb mit einer Injektionsvorrichtung in der Bereitschaftsposition;
- Fig. 13:: eine Seitenansicht gemäss Fig. 12 mit der Injektionsvorrichtung in der Setzposition;
- Fig. 14:: eine Seitenansicht gemäss Fig. 12 mit der Injektionsvorrichtung in der Endposition;
- Fig. 15:: eine äussere Schrägansicht eines Verschlussstücks eines Behälters;
- Fig. 16:: eine Ansicht auf eine Stirnseite des Verschlussstücks;
- Fig. 17:: eine Doppelschnittansicht der Schnittebenen, welche in Fig. 16 eingezeichnet sind;
- Fig. 18:: eine Doppelschnittansicht gemäss Fig. 17 mit einem Kolben einer Fördervorrichtung, welcher vollständig in einen Hubraum im Verschlussstück eingefahren ist;
- Fig. 19:: eine Doppelschnittansicht analog Fig. 18 mit vollständig ausgefahrenem Kolben;
- Fig. 20:: die Fördervorrichtung in einem ersten Takt eines Pumpzyklus;
- Fig. 21:: die Fördervorrichtung in einem zweiten Takt des Pumpzyklus;
- Fig. 22:: die Fördervorrichtung in einem dritten Takt des Pumpzyklus;
- Fig. 23:: die Fördervorrichtung in einem vierten Takt des Pumpzyklus;
- Fig. 24:: eine äussere Schrägansicht eines Behälters einer erfindungsgemässen Vorrichtung mit mehreren Innenräumen;
- Fig. 25:: eine Frontalansicht auf eine Stirnseite des Behälters der Fig. 24;
- Fig. 26:: eine äussere Schrägansicht eines Verschlussstücks des Behälters gemäss Fig. 24 von oben;
- Fig. 27:: eine äussere Schrägansicht eines Verschlussstücks des Behälters gemäss Fig. 24 von unten;
- Fig. 28:: eine äussere Schrägansicht eines weiteren Behälters einer erfindungsgemässen Vorrichtung mit mehreren Innenräumen und mehreren Fördervorrichtungen;
- Fig. 29:: eine äussere Schrägansicht des Verschlussstücks des Behälters gemäss Fig. 28 von oben;
- Fig. 30:: eine äussere Schrägansicht des Verschlussstücks des Behälters gemäss Fig. 28 von unten;
- Fig. 31-35:: verschiedene Funktionsschemata, wie die Innenräume eines Doppelkammerbehälters mit ein oder zwei Pumpen fluidmässig geschaltet sein können;
- Fig. 36:: eine Anordnung für eine erfindungsgemässe Vorrichtung, mit mehreren separaten Behältern;
- Fig. 37:: eine weitere Ausführungsform einer erfindungsgemässen Vorrichtung mit einem Linearantrieb als Setzantrieb in einer schrägen Teilansicht ohne Gehäuse;
- Fig. 38:: eine weitere Teilansicht auf die Vorrichtung der Fig. 37 in einer Bereitschaftsposition auf die Setzvorrichtung;
- Fig. 39:: eine Teilansicht gemäss Fig. 38 mit der Vorrichtung in einer Endposition auf die Setzvorrichtung;
- Fig. 40:: eine weitere Ausführungsform einer erfindungsgemässen Vorrichtung mit einem Linearantrieb als Setzantrieb in einer seitlichen Teilansicht ohne Gehäuse.

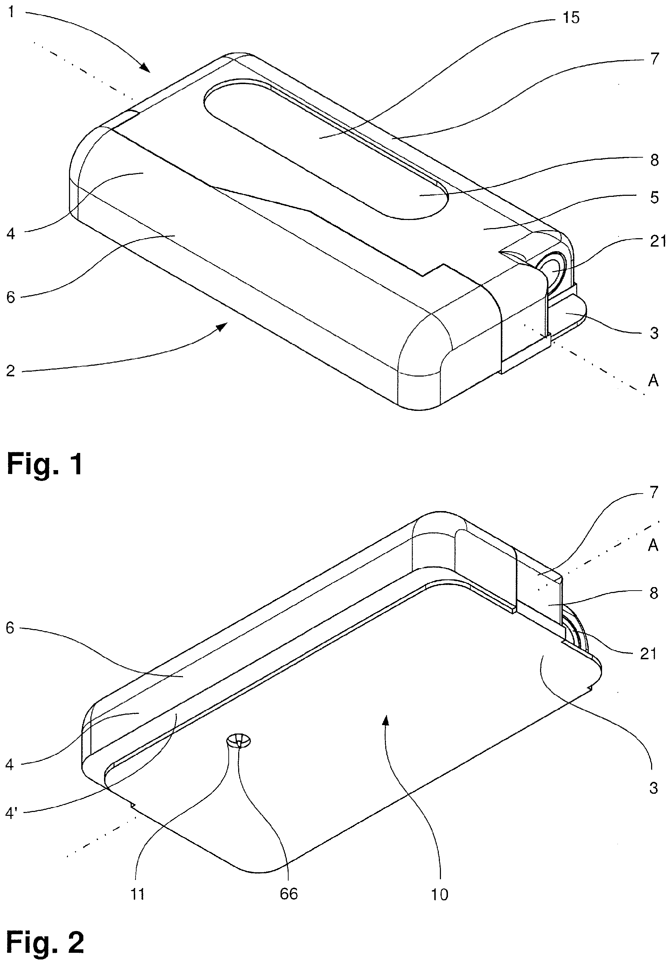

Figur 1 zeigt eine Schrägansicht von aussen auf eine erfindungsgemässe Vorrichtung 1 von oben her und Figur 2 von unten her. Figuren 1 und 2 sind im Folgenden gemeinsam beschrieben. Die Vorrichtung 1 ist als tragbares Abgabesystem zur subkutanen Verabreichung eines Fluids, insbesondere eines flüssigen Medikaments wie Insulin, ausgebildet.

Die Vorrichtung 1 umfasst ein Gehäuse 2, welches im Wesentlichen quaderförmig mit einer Längsachse A und an der Oberseite abgerundeten Kanten ausgebildet ist. Das Gehäuse 2 weist eine durchgehende Grundplatte 3 auf, auf welcher zwei Gehäuseschalen 4 und 5 angeordnet sind. Die Gehäuseschalen 4 und 5 stossen dabei weitgehend bündig längs einer Trennfläche aneinander und bilden gemeinsam mit der Grundplatte 3 das vollständige Gehäuse 2 der Vorrichtung 1. Die Gehäuseschalen 4 und 5 sind einem Antriebsmodul 6 - Gehäuseschale 4 - sowie einem Abgabemodul 7 - Gehäuseschale 5 - zugeordnet. Aufgrund der getrennten Gehäuseschalen 4 und 5 können das Antriebsmodul 6 und das Abgabemodul 7 voneinander getrennt werden. Das Abgabemodul 7 ist als Wegwerfmodul ausgebildet, während das Antriebsmodul 6 wieder verwendbar ist. Die Grundplatte 3 gehört zum Wegwerfmodul 7, wobei das Antriebsmodul 6 im zusammengesetzten Zustand der Vorrichtung 1 auf der Grundplatte 3 angeordnet ist.

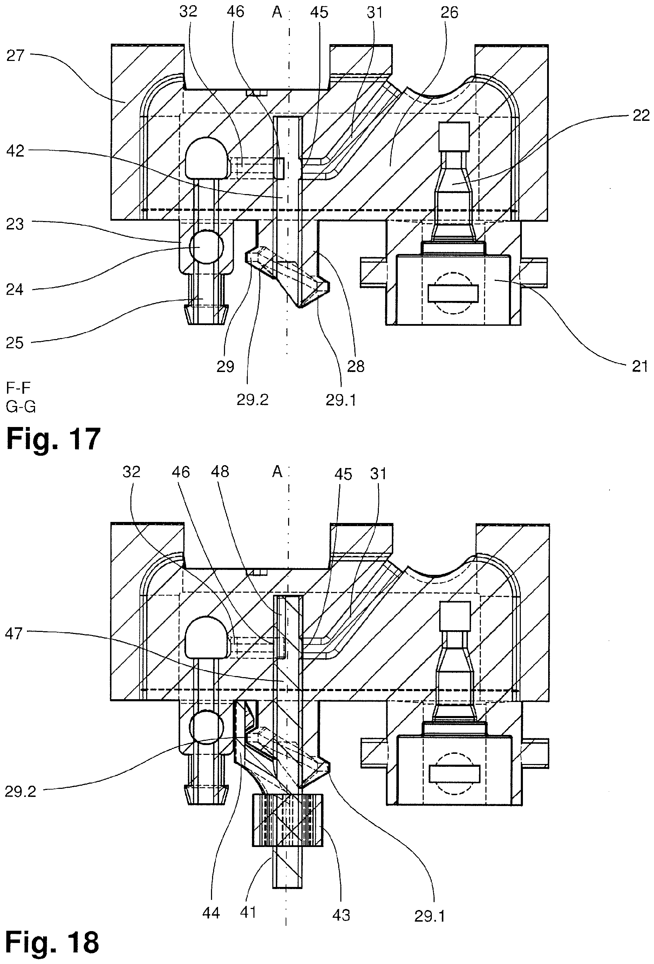

Die Gehäuseschale 5 weist ein Sichtfenster 8 auf, durch welches der Füllstand eines im Inneren des Gehäuses 2 angeordneten Behälters 15 überprüft werden kann. An einer Stirnseite an der Gehäuseschale 5 ist ein Füllport 21 ausgebildet, welcher mit einem Füllkanal 22 des Behälters 15 kommuniziert (siehe z.B. Fig. 17).

Aussenseitig an der Grundplatte 3 ist eine Kontaktfläche 10 ausgebildet, mit welcher die Vorrichtung 1 an die Hautoberfläche eines Patienten (nicht dargestellt) anlegbar ist. Die Kontaktfläche 10 ist vorliegend eben ausgebildet. Im Folgenden ist eine Anordnung der Grundplatte 3 sowie eine Richtung nach aussen, von der Kontaktfläche 10 weg, mit unten bezeichnet, während die Gehäuseschalen 4 und 5 oben angeordnet sind.

Die Kontaktfläche 10 kann zur Befestigung an der Hautoberfläche eine Klebeschicht aufweisen. Die Vorrichtung 1 kann somit an einer geeigneten Stelle am Körper des Patienten auf die Hautoberfläche geklebt werden und dort für längere Zeit verbleiben. An der Kontaktfläche 10 ist eine Setzöffnung 11 ausgebildet, durch welche eine Verweilkanüle 67 einer Injektionsvorrichtung 60 der Vorrichtung 1 zum Setzen ausfahrbar ist. Eine Öffnungsebene der Setzöffnung 11 liegt dabei in einer Ebene der Kontaktfläche 10.

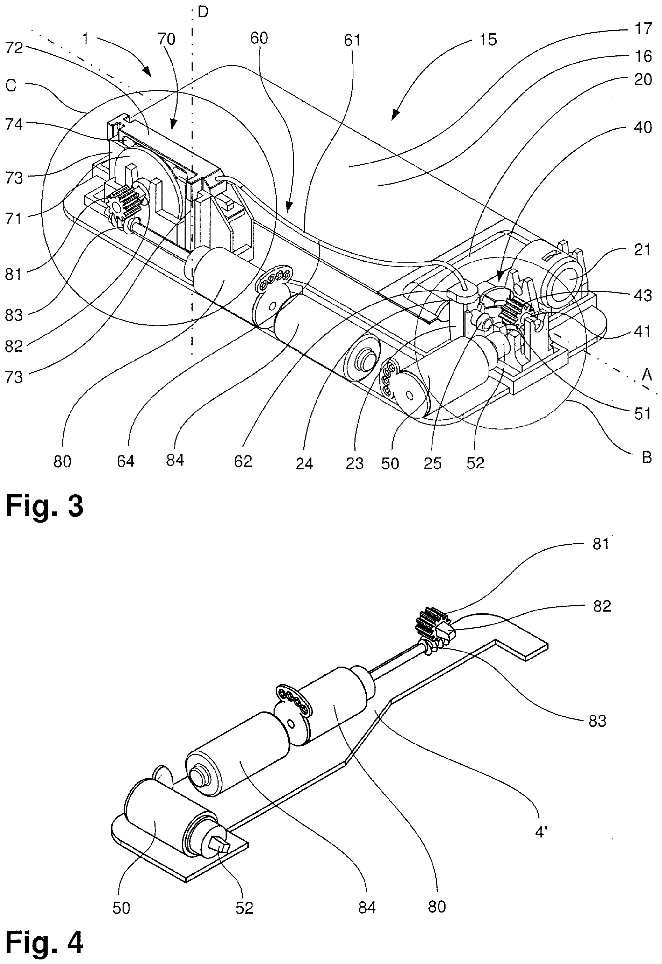

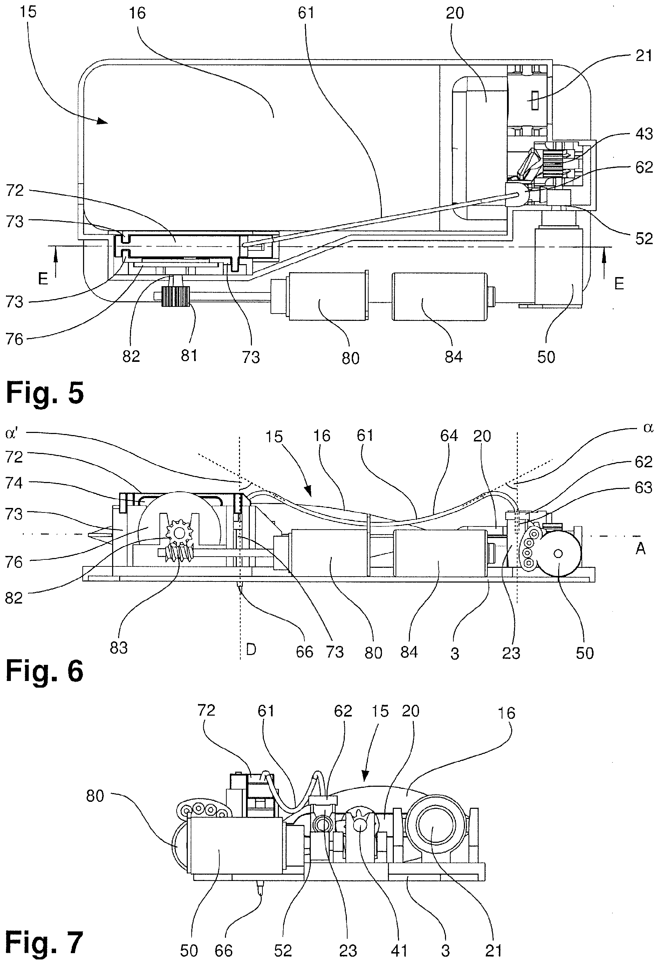

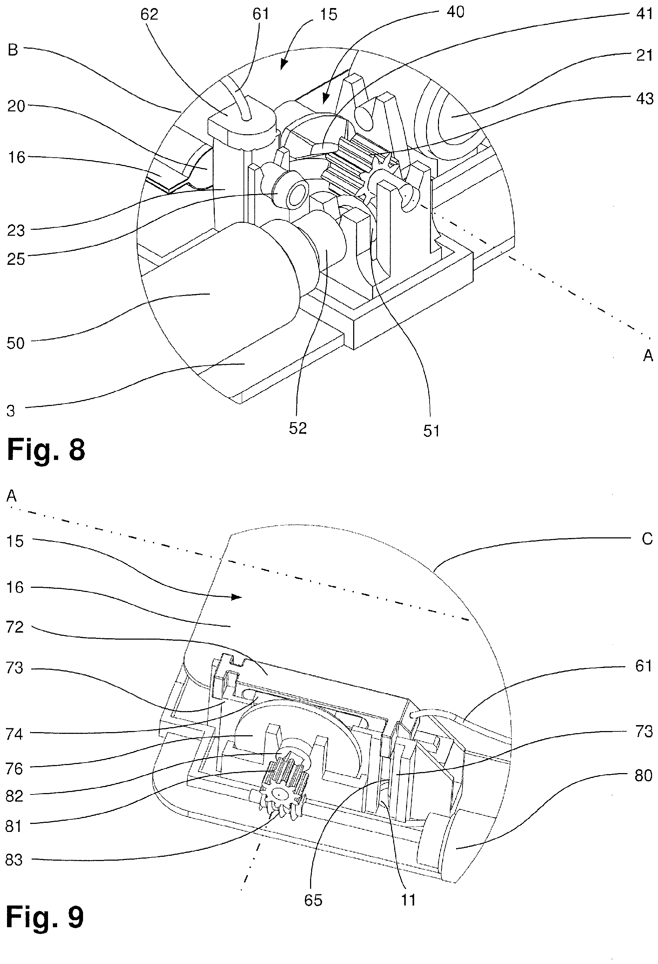

Figur 3 zeigt eine Schrägansicht der Vorrichtung 1 ohne die Gehäuseschalen 4 und 5. Figur 4 zeigt das Antriebsmodul 6 alleine ohne oberen Gehäuseteil. Figur 5 zeigt eine Draufsicht auf die Vorrichtung 1 von oben in Richtung senkrecht zur Grundplatte 3 bzw. der Kontaktfläche 10. Figur 6 zeigt eine antriebsseitige Seitenansicht und Fig. 7 eine Frontalansicht auf die Stirnseite mit dem Füllport 21. Figuren 8 und 9 zeigen Ausschnittsvergrösserungen eines Abgabebereichs B und eines Injektionsbereichs C gemäss Fig. 3. Figuren 3 bis 9 sind im Folgenden gemeinsam beschrieben.

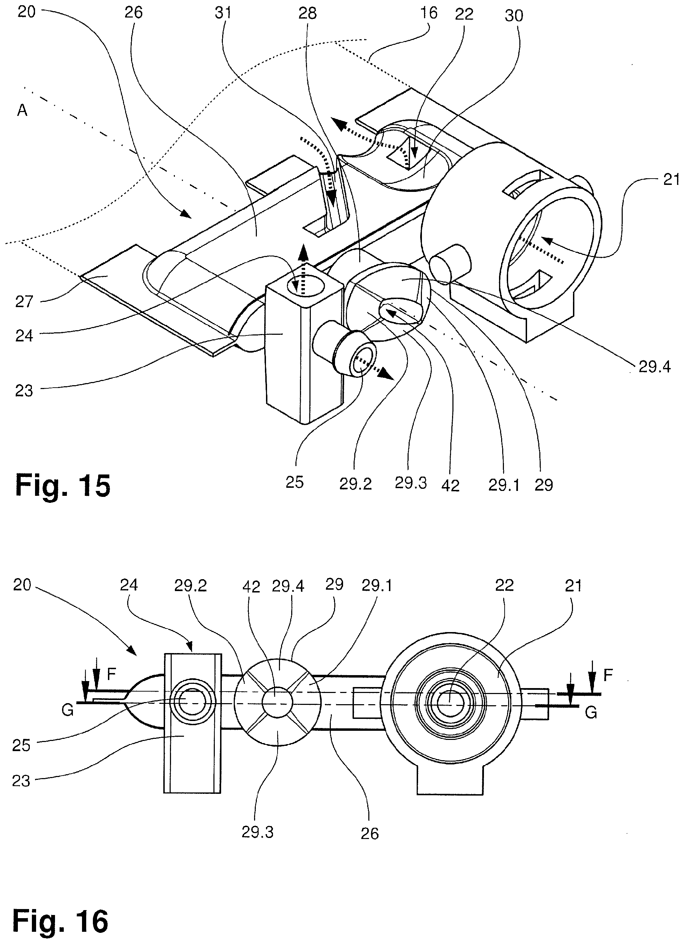

In Längsrichtung von A ist der Behälter 15 angeordnet, welcher vorliegend länglich als kollabierbarer Beutel ("Mini-Bag") ausgebildet ist und in dessen Innenraum 17 ein Fluid vorhanden ist. Das Fluid kann dabei ein Medikament umfassen, insbesondere Insulin. Der Behälter 15 umfasst ein Verschlussstück 20, welches fest mit einer flexiblen Behälterwand 16 verbunden ist. Die Behälterwand 16 umfasst zwei Lagen eines Folienmaterials, welche in den Randbereichen an einer Verbindungsnaht miteinander verbunden sind. An einem Längsende des Behälters 15 ist das Verschlussstück 20 zwischen den Folienlagen fest angeordnet. Die Folienlagen schliessen dabei fluiddicht an das Verschlussstück 20 an. Das Verschlussstück 20 ist z.B. anhand von Fig. 15 bis 17 näher beschrieben.

An einer Stirnseite eines Grundkörpers 26 des Verschlussstücks 20 ist der Füllport 21 angeordnet, welcher über den Füllkanal 22 mit dem von den Folienlagen gebildeten Innenraum 17 des Behälters 15 kommuniziert (siehe Fig. 15). Über den Füllport 21 kann der Innenraum des Behälters 15 erforderlichenfalls mit dem Fluid befüllt werden. Der Füllport 21 weist aussenseitig Haltestutzen auf, welche zur Halterung des Behälters 15 in der Vorrichtung 1 in Rastböcken der Grundplatte 3 verrastet sind (siehe z.B. Fig. 8) .

Ebenfalls stirnseitig am Verschlussstück 20 ist ein rohrförmiger Abgabeport 23 ausgebildet, welcher senkrecht zur Längsrichtung A und senkrecht zur Grundplatte 3 ausgerichtet ist. An einem oberen Rohrende weist der Abgabeport 23 eine Abgabeöffnung 24 auf (siehe z.B. Fig. 15). Eine Öffnung an einem unteren Rohrende des Abgabeports 23 ist von einem Verschlusszapfen der Grundplatte 3 verschlossen (nicht erkennbar). Zudem ist am Abgabeport 23 ein in Längsrichtung nach vorne weisender, vorliegend verschlossener, Anschlussstutzen 25 ausgebildet, an welchem gegebenenfalls z.B. ein Schlauch anschliessbar ist. Der Anschlussstutzen 25 ist zudem an einem Rastbock der Grundplatte 3 zur Halterung des Behälters 15 in der Vorrichtung 1 verrastet (siehe z.B. Fig. 8)

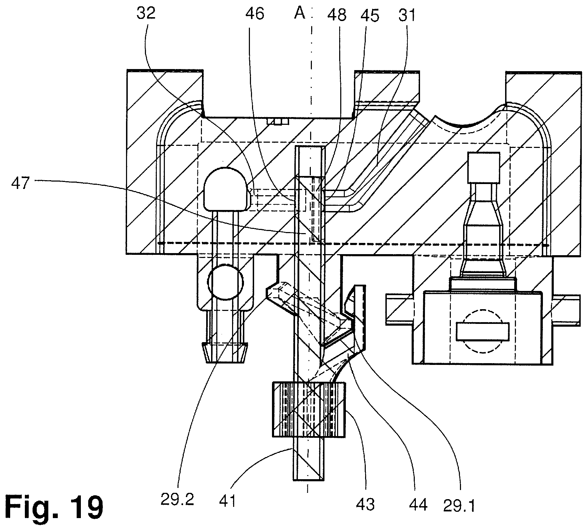

Zwischen Füllport 21 und Abgabeport 23 ist im Abgabebereich B (siehe hierzu Fig. 8) eine als Taumelkolbenpumpe 40 ausgebildete Fördervorrichtung am Verschlussstück 20 ausgebildet. Die Taumelkolbenpumpe 40 umfasst einen Kolben 41, welcher mit seiner Kolbenachse in Richtung von A in einen Hubraum 42 des Verschlussstücks 20 hineinragt (siehe hierzu z.B. Fig. 18 und 19). Der Kolben 41 weist in einem über das Verschlussstück 20 überstehenden Bereich ein Zahnrad 43 auf, welches koaxial zur Kolbenachse angeordnet ist. An einem freien Ende ist der Kolben 41 an einem Lagerbock der Grundplatte 3 gelagert, sodass das Zahnrad 43 zwischen Verschlussstück 20 und Lagerbock angeordnet ist.

In Richtung quer zur Kolbenachse ist eine Treibachse eines Drehantriebs 50 über ein Schneckenrad 51 an das Zahnrad 43 des Kolbens 41 gekoppelt. Das Schneckenrad 51 ist an Lagerböcken der Grundplatte 3 um eine Rotationsachse drehbar gelagert. Die Rotationsachse ist senkrecht zur Kolbenachse ausgerichtet. Der Drehantrieb 50 ist Teil eines Förderantriebs der Taumelkolbenpumpe 40. Das Schneckenrad 51 und das Zahnrad 43 bilden Teile eines Antriebsgetriebes des Förderantriebs.

Der Drehantrieb 50 ist mit seiner Triebachse über lösbare Kopplungsmittel 52 an das Schneckenrad 51 gekoppelt. Die Kopplungsmittel 52 sind bei jeweils einer Öffnung in der Wand der Gehäuseteile 4 und 5 (nicht dargestellt) angeordnet und umfassen treibachsenseitig, d.h. am Antriebsmodul 6, einen axial angeordneten konischen Vierkantzapfen sowie schneckenradseitig, d.h. am Abgabemodul 7, eine komplementäre Buchse für den Zapfen. Der Drehantrieb 50 kann somit im Antriebsmodul 6 angeordnet sein, während das Schneckenrad 51 im Abgabemodul 7 angeordnet ist. Beim Zusammensetzen der Module 6 und 7 können die Kopplungsmittel 52 in einer vorgegebenen Eingriffsrichtung zum Eingriff gebracht werden.

In die Abgabeöffnung 24 des Abgabeports 23 ist ein Anschlusszapfen 62 einer Injektionsvorrichtung 60 eingesetzt. Der Anschlusszapfen 62 ist an einer Hohlnadel 61 angeordnet und stellt eine fluiddichte Verbindung zwischen der Hohlnadel 61 und der Abgabeöffnung 24 her. Ein proximaler Endbereich 63 der Hohlnadel 61, an welchem der Anschlusszapfen 62 angeordnet ist, ist weitgehend senkrecht zur Grundplatte 3 ausgerichtet und mit dem Anschlusszapfen 62 in der Abgabeöffnung 24 am Abgabeport 23 und damit ortsfest in der Vorrichtung 1 fixiert (siehe insbesondere z.B. Fig. 6).

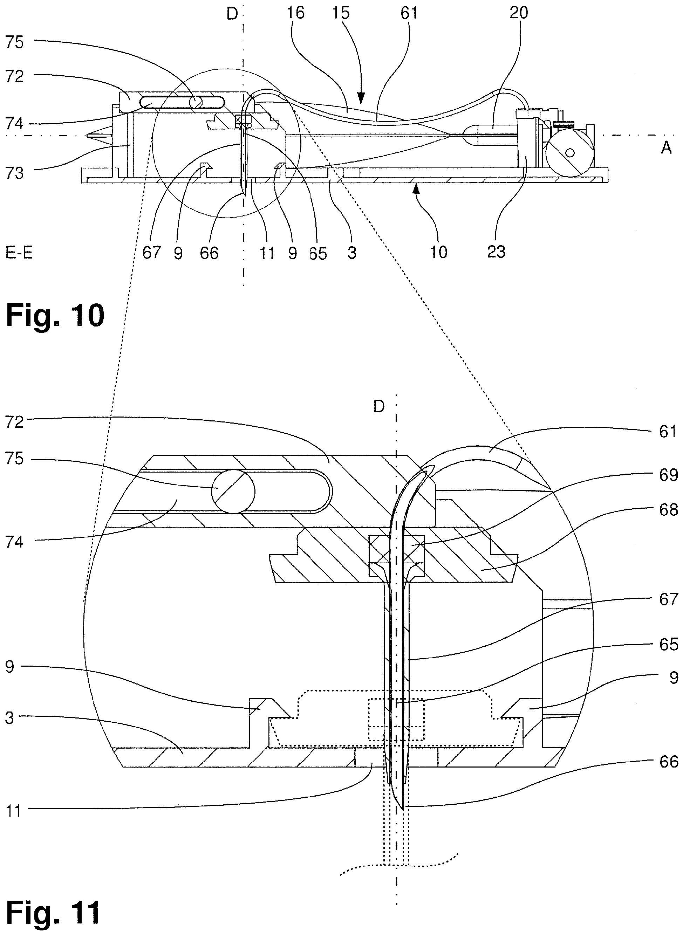

Vom proximalen Endbereich 63 erstreckt sich die Hohlnadel 61 mit einem Mittelbereich 64 von der Abgabeöffnung 24, gegenüber der Längsrichtung A geringfügig geneigt, aber im Wesentlichen in Längsrichtung von A in der Vorrichtung 1 nach hinten zum Injektionsbereich C (siehe hierzu Fig. 9). Der Injektionsbereich C ist in Richtung quer zu A neben dem Behälter 15 angeordnet. Im Injektionsbereich C ist eine Setzvorrichtung 70 für die Verweilkanüle 67 ausgebildet (siehe z.B. Fig. 10 und 11). Die Setzvorrichtung 70 umfasst eine Führungsvorrichtung 71 mit einem Läufer 72, an welchem ein distaler Endbereich 65 (siehe z.B. auch Fig. 10 und 11) der Hohlnadel 61 fest angebracht ist. Der distale Endbereich 65 ist als Einstechbereich ausgebildet. Der Einstechbereich 65 ist im Wesentlichen parallel zum proximalen Endbereich 63, d.h. ebenfalls weitgehend senkrecht zur Grundplatte 3, ausgerichtet. Die Hohlnadel 61 ist bevorzugt als Stahlkanüle ausgebildet.

Dabei ist der Mittelbereich 64 zur Grundplatte 3 hin gewölbt, d.h. gekrümmt ausgebildet, sodass die Hohlnadel 61 im Mittelbereich 64 näher an der Grundplatte 3 angeordnet ist, als an der Abgabeöffnung 24 oder im Injektionsbereich C. Mit anderen Worten "hängt" die Hohlnadel 61 im Mittelbereich 64 zwischen dem proximalen Endbereich 63 und dem Einstechbereich 65 zur Grundplatte 3 hin durch.

Der Läufer 72 ist an Führungsschienen 73 der Führungsvorrichtung 71 in einer Verschieberichtung D senkrecht zur Grundplatte 3 verschiebbar gelagert. Die Führungsschienen 73 sind an der Grundplatte 3 fest angebracht, insbesondere angeformt. In der Darstellung der Fig. 3 bis 6 ist der Läufer 72 vollständig von der Grundplatte 3 weg verschoben. Der am Läufer 72 fest angeordnete Einstechbereich 65 ist somit im Wesentlichen vollständig durch die Setzöffnung 11 in das Gehäuse 2 eingezogen. Eine Einstechspitze 66 am distalen Ende des Einstechbereichs 65 kann dabei durch die Setzöffnung 11 nach aussen ragen (siehe z.B. Fig. 2). Ist die erwähnte Klebeschicht an der Kontaktfläche 10 vorhanden, ist die Einstechspitze 66 von der Klebeschicht geschützt.

Zwischen dem proximalen Endbereich 63 und einem benachbarten Abschnitt des Mittelbereichs 64 ist ein Winkel α eingeschlossen. Der Winkel α ist aufgrund der Krümmung des Mittelbereichs 64 kleiner als 90 Grad. Ebenso ist zwischen dem Einstechbereich 65 und einem benachbarten Abschnitt des Mittelbereichs 64 ein Winkel α' kleiner als 90 Grad eingeschlossen. Zwischen dem proximalen Endbereich 63 und dem benachbarten Abschnitt des Mittelbereichs 64 sowie zwischen dem Einstechbereich 63 und dem zu diesem benachbarten Abschnitt des Mittelbereichs 64 ist die Hohlnadel 61 in einem Übergangsbereich gegensinnig zum Mittelbereich 64 gekrümmt.

Im Läufer 72 ist senkrecht zur Verschieberichtung D eine Führungsnut 74 ausgebildet. In die Führungsnut 74 greift ein Nocken 75 einer Rotationsscheibe 76 eines Setzgetriebes ein, über welches ein Setzantrieb 80 mit einer Treibachse an die Führungsvorrichtung 71 gekoppelt ist. Die Rotationsscheibe 76 ist an Lagerböcken der Grundplatte 3 um eine Rotationsachse quer zur Verschieberichtung D und zur Längsrichtung der Führungsnut 74 rotierbar gelagert. Der Nocken 75 ist exzentrisch bezüglich der Rotationsachse an der Rotationsscheibe 76 angeordnet und greift in die Führungsnut 74 ein. Bei einer Drehung der Rotationsscheibe 76 wird der Läufer 74 aufgrund des Nockens 75 in den Führungsschienen 73 in der Verschieberichtung D verschoben.

Die Rotationsscheibe 76 ist über Kopplungsmittel 82 an ein Zahnrad 81 gekoppelt. Das Zahnrad 81 wiederum ist über ein Schneckenrad 83 an die Treibachse des Setzantriebs 80 gekoppelt. Die Treibachse des Setzantriebs 80 ist senkrecht zur Rotationsachse der Rotationsscheibe 76, d.h. insbesondere parallel zur Längsrichtung A angeordnet. Die Kopplungsmittel 82 sind dabei analog den Kopplungsmitteln 52 ausgebildet, wobei der Vierkantzapfen zahnradseitig und die Buchse rotationsscheibenseitig angeordnet ist. Die Kopplungsmittel 82 sind ebenfalls bei jeweils einer Öffnung in der Wand der Gehäuseteile 4 und 5 angeordnet. Insbesondere sind die Eingriffsrichtungen der Kopplungsmittel 52 und 82 parallel ausgerichtet, sodass beim Zusammensetzen der Module 6 und 7 in Eingriffsrichtung gleichzeitig der Setzantrieb 80 mit der Setzvorrichtung 70 und der Drehantrieb 50 mit der Taumelkolbenpumpe 40 koppelbar ist.

Der Setzantrieb 80 ist zusammen mit dem Zahnrad 81 und dem Schneckenrad 83 im Antriebsmodul 6 angeordnet. In der Ansicht der Fig. 4 ist nur eine Bodenplatte 4' des Gehäuseteils 4 dargestellt. Eine Lagerung des Zahnrads 81 und des Schneckenrads 83 sowie allfällige weitere Lager- und/oder Befestigungsmittel zur Halterung der Komponenten sind in Fig. 4 nicht dargestellt.

Das Antriebsmodul 6 kann zudem eine elektrische Spannungsquelle wie z.B. eine Batterie 84 umfassen, welche den Setzantrieb 80 sowie den Drehantrieb 50 mit elektrischer Energie versorgt. Zudem kann eine (nicht dargestellte) Steuerungselektronik zur Steuerung der Antriebe 50 und 80 sowie Kommunikationsmittel z.B. zur kabellosen oder kabelgestützten Anbindung einer Fernbedienung im Antriebsmodul 6 vorhanden sein. Ebenso können Schnittstellen zum Datenaustausch mit einem externen Computer und/oder zur Versorgung mit elektrischer Energie aus einer externen Quelle vorgesehen sein. Selbstverständlich können auch Überwachungsvorrichtungen vorhanden sein, welche die Antriebe sowie die Getriebe auf ihre korrekte Funktion überwachen.

Figur 10 zeigt eine Schnittansicht in einer Schnittebene E (siehe Fig. 5), welche parallel zu A ist und die Einstichrichtung D umfasst, d.h. senkrecht zur Grundplatte 3 ausgerichtet ist. Die Ebene E geht durch den Einstechbereich 65 der Hohlnadel 61. Figur 11 zeigt eine Ausschnittsansicht der Schnittansicht der Fig. 10 im Bereich des Einstechbereichs 65. Figuren 10 und 11 sind im Folgenden gemeinsam beschrieben.

Die Hohlnadel 61 ist im Übergangsbereich zwischen Einstechbereich 65 und Mittelbereich 64 am Läufer 72 befestigt. Die Hohlnadel 61 kann dabei direkt vom Läufer 72 umspritzt oder in diesem eingeklebt sein. Der Einstechbereich 65 ist in der Einstechrichtung D und somit senkrecht zur Grundplatte 3 ausgerichtet. Die Einstechspitze 66 ragt geringfügig durch die Setzöffnung 11 hindurch. Die Setzöffnung 11 weist dabei eine Öffnungsfläche auf, welche deutlich grösser ist, als ein äusserer Querschnitt der Verweilkanüle 67. Auf diese Weise ist sichergestellt, dass die Verweilkanüle 67 berührungsfrei durch die Setzöffnung 11 ausgefahren werden kann.

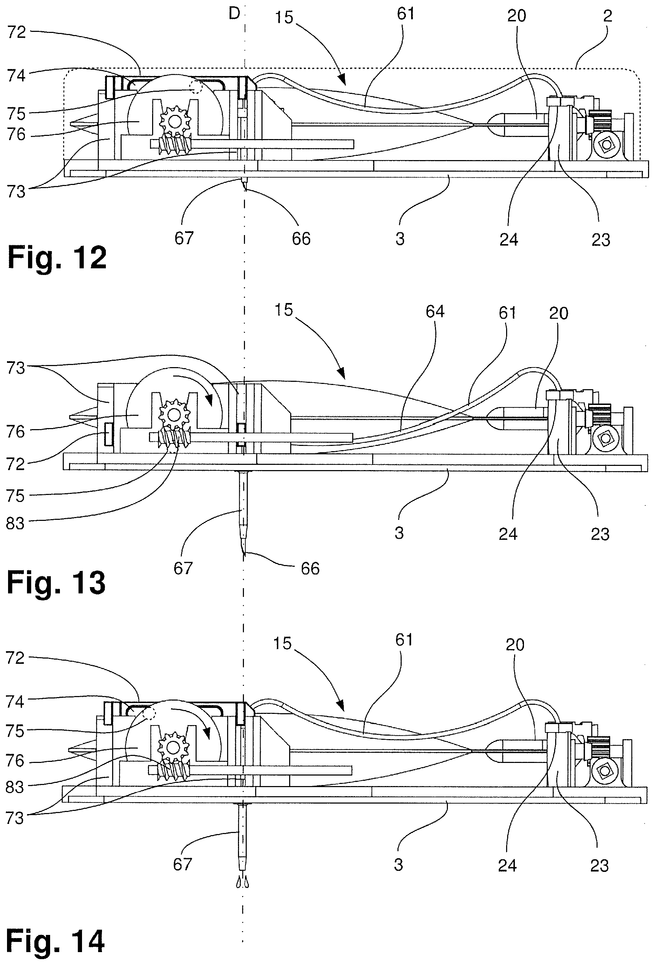

Der Einstechbereich 65 ist von der Verweilkanüle 67 ummantelt. Die Verweilkanüle 67 ist dabei aus einem flexiblen bioverträglichen Material gefertigt, welches für längere Zeit im Körper eines Patienten verbleiben kann. Die Verweilkanüle 67 sitzt dabei fest genug auf dem Einstechbereich 65, dass sie beim Übergang aus der Bereitschaftsposition in die Setzposition beim Penetrieren der Haut des Patienten vom Einstechbereich 65 mitgenommen wird. Dabei sitzt sie aber lose genug, dass die Verweilkanüle 67 beim Zurückziehen des Einstechbereichs 65 in die Endposition vom Einstechbereich 65 abgezogen werden kann (siehe hierzu auch Fig. 12 bis 14).

Hierzu umfasst die Grundplatte 3 ein Rastmittel 9, welches bei der Setzöffnung 11 angeordnet ist. Die Verweilkanüle 67 ist mit einem entsprechend komplementär ausgebildeten Rastmittel 68 versehen, welches mit dem Rastmittel 9 zum verrastenden Eingriff bringbar ist, wenn der Einstechbereich 65 zusammen mit der Verweilkanüle 67 durch die Setzöffnung 11 in die Setzposition ausgefahren ist. Vorliegend umfasst das Rastmittel 9 einen um die Setzöffnung 11 angeordneten Rastkranz und das Rastmittel 68 der Verweilkanüle 67 einen an einem proximalen Längsende der Verweilkanüle 67 angeordneten Rastkragen. Der Rastkragen 68 verrastet selbsttätig im Rastkranz 9, wenn die Verweilkanüle 67 in die Setzposition gelangt (in Fig. 11 gestrichelt angedeutet).

Der Rastkragen 68 kann dabei aus einem anderen, beispielsweise härteren Material als die Verweilkanüle 67 gefertigt sein. Typischerweise ist die Verweilkanüle 67 mit ihrem proximalen Endabschnitt in einem Durchtritt des Rastkragens 68 festgeklemmt. Zusätzlich ist ein Dichtelement 69 im Durchtritt des Rastkragens 68 vorgesehen, welches als Gleitdichtung zwischen der Verweilkanüle 67 und der Hohlnadel 61 ausgebildet ist. Die Gleitdichtung 69 stellt sicher, dass in allen vorgesehenen relativen Positionen der Hohlnadel 61 und der Verweilkanüle 67 eine fluiddichte Verbindung zwischen der Hohlnadel 61 und der Verweilkanüle 67 besteht.

Der Rastkragen 68 liegt dabei in der Bereitschaftsposition bevorzugt am Läufer 72 der Führungsvorrichtung 71 an, sodass eine Kraftwirkung vom Läufer 72 beim Einstechen auch direkt auf den Rastkragen 68 bzw. die Verweilkanüle 67 wirkt. Zudem wird der Rastkragen 68 beim Erreichen der Setzposition vom Läufer 72 zuverlässig in den Rastkranz 9 gedrückt.

Figuren 12 bis 14 zeigen Seitenansichten der Vorrichtung 1 weitgehend entsprechend Fig. 6, wobei Fig. 12 die Injektionsvorrichtung 60 in der Bereitschaftsstellung, Fig. 13 in der Setzposition und Fig. 14 in der Endposition zeigt.

Figur 12 entspricht im Wesentlichen der Darstellung der Fig. 6, wobei der Setzantrieb 80 sowie die Batterie 84 und der Drehantrieb 50 zur besseren Übersichtlichkeit ausgeblendet sind. Der Läufer 72 ist in den Führungsschienen 73 so weit wie möglich von der Grundplatte 3 weg verschoben. Der Einstechbereich 65 der Hohlnadel 61 ist weitgehend vollständig im Gehäuse 2 (nicht dargestellt) angeordnet. Die Einstechspitze 66 ragt durch die Setzöffnung 11 geringfügig über die Kontaktfläche 10 hinaus.

In Fig. 13 ist der Läufer 72 vollständig zur Grundplatte 3 hin verschoben. Die Verschiebung wird durch Rotation der Rotationsscheibe 76 in einer Setzrichtung (siehe Pfeil) bewirkt, wodurch der daran angeordnete Nocken 75 zur Grundplatte 3 hin abgesenkt wird. Dabei nimmt der Nocken 75 den Läufer 72 mit, während er in Längsführung 74 quer zur Einstechrichtung bzw. der Verschieberichtung D der Führungsvorrichtung 71 verschoben wird.

Mit der Verschiebung des Läufers 72 ist der fest daran angebrachte Einstechbereich 65 der Hohlnadel 61 durch die Setzöffnung 11 ausgefahren. Die Verweilkanüle 67 ist damit in die Setzposition gebracht, wobei der Rastkragen 68 im Rastkranz 9 verrastet ist (nicht sichtbar). Aufgrund der Linearverschiebung des Läufers 72 ist die Distanz zur fest im Gehäuse 2 angeordneten Abgabeöffnung 24 grösser, als in der Bereitschaftsposition. Der damit erforderliche Längenausgleich der Hohlnadel 61 wird durch den Mittelbereich 64 der Hohlnadel 61 erreicht, welcher in der Setzposition gegenüber der Bereitschaftsposition gestreckt ist, d.h. eine geringere Krümmung aufweist. Aufgrund der Führungsvorrichtung 71 kann die Hohlnadel 61 eine vergleichsweise grosse Steifigkeit aufweisen und z.B. aus Stahl gefertigt sein, ohne dass die Einstechbewegung durch die zum Strecken erforderliche Kraft gestört wird. Die Führungsvorrichtung 71 stellt sicher, dass der Einstechbereich 65 die Haut des Patienten geradlinig penetriert.

Figur 14 zeigt die Injektionsvorrichtung 60 in der Endposition. Durch weitere Rotation der Rotationsscheibe 76 in Setzrichtung wird der Läufer 72 über den Nocken 75 wieder zurück, von der Grundplatte 3 weg verschoben. Die Stellung des Läufers 72 in der Endposition entspricht der Stellung in der Bereitschaftsposition. Dabei wird auch der fest mit dem Läufer 72 verbundene Einstechbereich 65 der Hohlnadel 61 durch die Setzöffnung 11 in das Gehäuse 2 zurückgezogen. Die mit dem Rastkragen 68 im Rastkranz 9 der Grundplatte verrastete Verweilkanüle 67 verbleibt dabei in der Setzposition. Die Verweilkanüle wird 67 somit zumindest teilweise vom Einstechbereich 65 abgezogen. Ein Endbereich an der Einstechspitze 66 des Einstechbereichs 65 ragt in der Endposition in die Verweilkanüle 67 hinein (nicht sichtbar). Insbesondere liegt das Dichtelement 69 aussenseitig fluiddicht an dem Endbereich an der Einstechspitz 66 an. Auf diese Weise kann die gesetzte Verweilkanüle 67 über die Hohlnadel 61 von der Abgabeöffnung 24 mit dem Fluid aus dem Behälter 15 beschickt werden.

Die Rotationsscheibe 76 ist aufgrund des Schneckenrads 83 jeweils in einer einmal eingenommenen Rotationsstellung festgestellt. Über den Nocken 75 ist somit auch der Läufer 72 festgestellt, sodass ein versehentliches erneutes Ausfahren bzw. Einstechen der Hohlnadel 61 zuverlässig verhindert ist. Das Schneckenrad 83 bildet somit zusammen mit der Rotationsscheibe 76 einen Teil eines selbst arretierenden Setzgetriebes der Setzvorrichtung 70.

Figur 15 zeigt eine äussere Schrägansicht des Verschlussstücks 20. Figur 16 zeigt eine Ansicht längs A auf die Stirnseite des Verschlussstücks 20 und Fig. 17 eine Doppelschnittansicht gemäss den in Fig. 16 eingezeichneten Schnittebenen F und G. Figuren 15 bis 17 sind im Folgenden gemeinsam beschrieben.

Das Verschlussstück 20 ist vorliegend als einstückiges Spritzgussteil ausgebildet. Der Grundkörper 26 hat einen in Richtung quer zu A abgeflachten Querschnitt, welcher zu den Rändern hin in einem teilweise umlaufenden Flansch 27 ausläuft. Der Flansch 27 dient dabei zum Zusammenführen der Folienlagen, welche die Behälterwand 16 bilden und zwischen welchen das Verschlussstück 20 angeordnet ist. Das Verschlussstück 20 umfasst einen Stutzen 28, welcher sich von der Stirnseite des Verschlussstücks 20 in Richtung von A nach vorne erstreckt. Der Hubraum 42 der Taumelkolbenpumpe 40 ist als nach vorne offener, kreiszylindrischer Hohlraum im Stutzen 28 ausgebildet und reicht bis in den Grundkörper 26 hinein. Der Hubraum 42 ist vorliegend koaxial mit A ausgebildet.

Eine Stirnseite des Stutzens 28 ist bezüglich A abgeschrägt ausgebildet und weist einen bezüglich A weitgehend radial nach aussen auskragenden Flansch 29 auf. Der Flansch 29 ist als Steuerfläche für den Kolben 41 ausgebildet (siehe hierzu z.B. Fig. 17). Die Steuerfläche 29 weist in Richtung von A umfangsseitig unterschiedliche Abstände von der Stirnseite des Verschlussstücks 20 auf, sodass ein an der Steuerfläche 29 geführter Läufer 44 (siehe z.B. Fig. 18 und 19) des Kolbens 41 den Kolben 41 in eine Hubbewegung zwingt, wenn dieser um A rotiert wird. Je nach Ausbildung z.B. des Kolbens 41 und des Hubraums 42 kann die Steuerfläche 29 Abschnitte mit unterschiedlichen Steigungen aufweisen, sodass ein aufgrund einer Drehung erzeugbarer Hub sich je nach Drehstellung des Kolbens 41 unterscheiden kann. Vorliegend umfasst die Steuerfläche 29 vier Abschnitte, von welchen zwei bezüglich A gegenüberliegende Abschnitte 29.1 und 29.2 in unterschiedlichen Längspositionen bezüglich A senkrecht ausgebildet sind und die beiden weiteren Abschnitte 29.3 und 29.4 bezüglich A geneigt sind und an die Abschnitte 29.1 und 29.2 anschliessen.

Im Grundkörper 26 ist der Füllkanal 22 ausgebildet, welcher mit dem Innenraum 17 des Behälters 15 kommuniziert. Der Füllkanal 22 tritt dabei an der Stirnseite am Füllport 21 aus. Der Füllport 21 weist einen kreiszylinderförmigen Rohrabschnitt als Kupplungselement zum Ankoppeln einer Fluidquelle auf. An einer Unterseite des Rohrabschnitts ist eine Auflagefläche ausgebildet, mit welcher der Füllport 21 an der Grundplatte 3 des Gehäuses 2 abstützbar und gegebenenfalls befestigbar ist.

Zum Innenraum des Behälters 15 hin mündet der Füllkanal 22 an einer im Grundkörper 26 ausgebildeten Mulde 30 des Verschlussstücks 20. Die Mulde 30 stellt sicher, dass ein Fluid durch den Füllkanal 22 in den Innenraum 17 eintreten kann. Gleichzeitig bildet die Mulde 30 eine Auflagefläche, gegen welche eine der Folienlagen der Behälterwand 16, welche die Mulde 30 überdeckt, zum fluiddichten Verschliessen des Füllkanals 22 von aussen her gepresst werden kann. Hierzu kann beispielsweise am Gehäuse 2 ein innen liegender Vorsprung ausgebildet sein (nicht dargestellt), welcher derart ausgebildet und bemessen ist, dass die Folienlage im betriebsbereiten Zustand die Folie dichtend in die Mulde 30 presst. Alternativ kann im Füllkanal 22 ein Schnabelventil ausgebildet sein (nicht dargestellt), welches einen Rückfluss des Fluids aus dem Innenraum 17 durch den Füllkanal 22 verhindert.

Weiter ist ein Entnahmekanal 31 im Grundkörper 26 ausgebildet, welcher ebenfalls mit dem Innenraum des Behälters 15 kommuniziert. Der Entnahmekanal 31 mündet direkt neben der Mulde 30 in den Innenraum 17 des Behälters 15 und ist von derselben Folienlage überdeckt, wie die Mulde 30. Der Entnahmekanal 31 erstreckt sich zum Hubraum 42 hin und mündet an einer in Richtung A länglichen Zuführungsöffnung 45 in den Hubraum 42 (siehe z.B. Fig. 17) .

An einer Abführungsöffnung 46 (siehe z.B. Fig. 17) mündet ein im Inneren des Grundkörpers 26 geführter Abgabekanal 32 in den Hubraum 42. Zuführungsöffnung 45 und Abgabeöffnung 46 sind bezüglich der Hubraumachse, d.h. vorliegend bezüglich der Längsachse A, weitgehend gegenüberliegend an einer mantelseitigen Innenwand des Hubraums 42 angeordnet.

Der Abgabekanal 32 erstreckt sich zum rohrförmigen Abgabeport 23, welcher an der Stirnseite des Verschlussstücks 20 ausgebildet ist. Der Abgabekanal 32 kommuniziert mit einem Innenraum des Abgabeports und der daran ausgebildeten Abgabeöffnung 24 und Anschlussöffnung 25.

Figuren 18 und 19 zeigen Doppelschnittansichten analog der Fig. 17 des Verschlussstücks 20 mit daran angeordnetem Kolben 41. In Fig. 18 ist der Kolben 41 vollständig in den Hubraum 42 eingefahren und in Fig. 19 vollständig ausgefahren.

Der Kolben 41 weist einen weitgehend kreiszylindrischen Grundkörper 47 auf, dessen Aussenmasse mit einer Beweglichkeitstoleranz den Innenmassen des Hubraums 42 entsprechen. An einem aus dem Hubraum 42 hinaus ragenden Abschnitt ist das Zahnrad 43 ausgebildet. Am Grundkörper 47 ist der Läufer 44 angeordnet, welcher die Steuerfläche 29 in Richtung von A beidseitig umgreift. Auf diese Weise wird eine Zwangskopplung zwischen der Drehbewegung und der Hubbewegung des Kolbens 41 erreicht.

An einem im Hubraum 42 angeordneten Endabschnitt des Kolbens ist mantelseitig eine Längsrille 48 ausgebildet. Die Längsrille 28 ist dabei je nach Drehstellung des Kolbens 41 mit der Zuführungsöffnung 45, der Abführungsöffnung 46 oder mit keiner der beiden Öffnungen in Fluidkommunikation. Somit können im Wesentlichen vier Pumptakte unterschieden werden, welche den vier Abschnitten 29.1 bis 29.4 der Steuerfläche 29 entsprechen:
Auf den ansteigenden oder absteigenden Abschnitten 29.3 bzw. 29.4 ist die Längsrille 48 entweder mit der Abführungsöffnung 46 oder der Zuführungsöffnung 45 in Fluidkommunikation. Durch die Hubbewegung wirkt der Kolben 41 entweder als Verdränger, welcher ein Fluid im Hubraum 42 durch die Abführungsöffnung 46 in den Abgabekanal 32 drängt oder als Saugkolben, welcher das Fluid durch den Entnahmekanal 31 in den Hubraum 42 ansaugt. Bei den Abschnitten 29.1 und 29.2 führt der Kolben 41 keine Hubbewegung aus, sodass das vom Kolben 41 definierte Volumen im Hubraum 42 konstant bleibt. In den durch die Abschnitte 29.1 und 29.2 definierten Takten wird die Längsrille 48 von der Zuführungsöffnung 45 zur Abführungsöffnung 46 rotiert, oder umgekehrt.

In Fig. 18 befindet sich der Läufer 44 auf dem Abschnitt 29.2 der Steuerfläche 29 und in Fig. 19 auf dem in Richtung A weiter vom Grundkörper 26 des Verschlussstücks 20 entfernten Abschnitt 29.1.

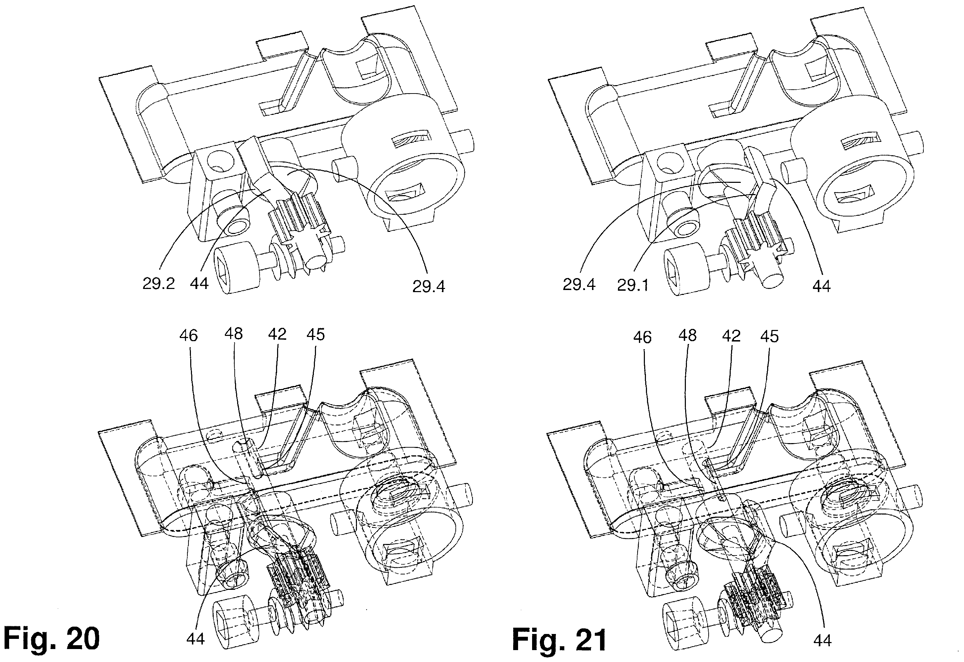

Figuren 20 bis 23 zeigen die vier Arbeitstakte der Taumelkolbenpumpe 40 in jeweils einer äusseren Schrägansicht und einer Transparentansicht.

Figur 20 zeigt den ersten Takt beim Übergang des Läufers 44 vom Abschnitt 29.2 auf den ansteigenden Abschnitt 29.4. Der Kolben 41 ist an dieser Stelle noch vollständig in den Hubraum 42 eingefahren. Dabei gerät die Längsrille 48 mit der Zuführungsöffnung 45 in Fluidkommunikation.

Figur 21 zeigt den zweiten Takt, während welchem der Kolben 41 aufgrund des Abschnitts 29.4 aus dem Hubraum 42 gezwungen wird. Dabei wird aufgrund des so entstehenden Unterdrucks im Hubraum 42 das Fluid vom Kolben 41 über den Entnahmekanal 31 in den Hubraum 42 gesogen.

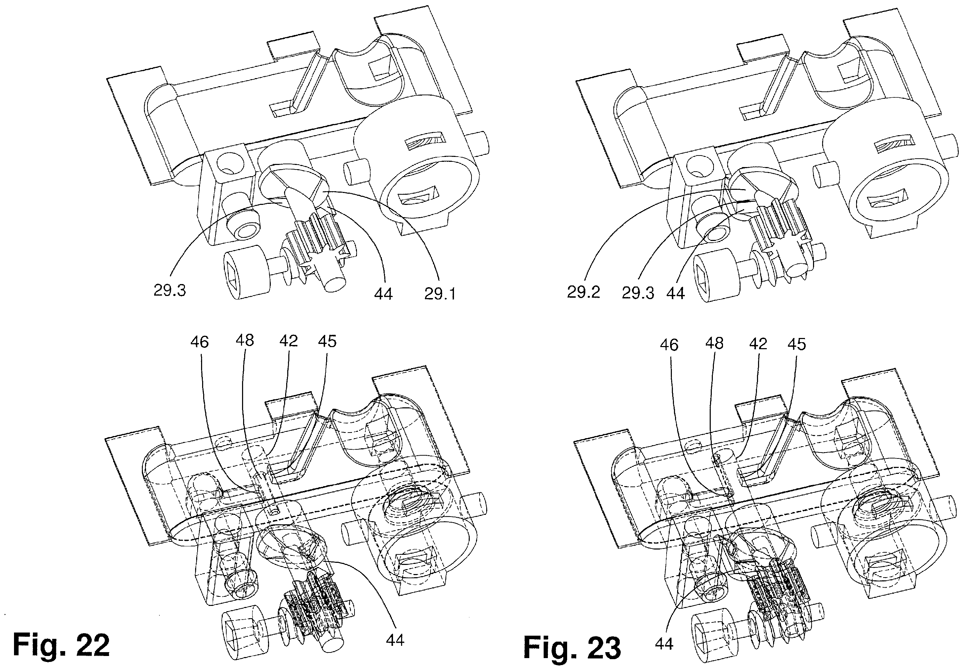

Figur 22 zeigt den dritten Takt beim Übergang des Läufers 44 vom Abschnitt 29.1 auf den absteigenden Abschnitt 29.3. Der Kolben 41 ist an dieser Stelle noch vollständig aus dem Hubraum 42 ausgefahren. Dabei gerät die Längsrille 48 mit der Abführungsöffnung 46 in Fluidkommunikation.

Figur 23 zeigt schliesslich den vierten und letzten Takt, während welchem der Kolben 41 aufgrund des Abschnitts 29.3 in den Hubraum 42 gezwungen wird. Dabei wird aufgrund des so entstehenden Überdrucks im Hubraum 42 das Fluid vom Kolben 41 über den Abgabekanal 32 aus dem Hubraum 42 gedrängt.

Der Arbeitszyklus der Taumelkolbenpumpe 40 beginnt nach dem vierten Takt wieder von vorne mit dem ersten Takt.

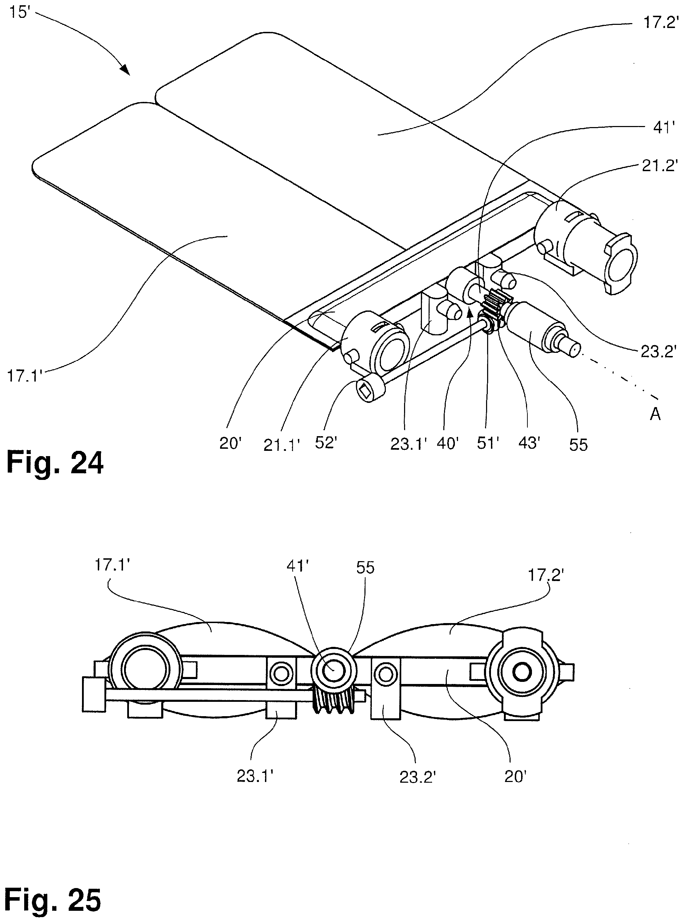

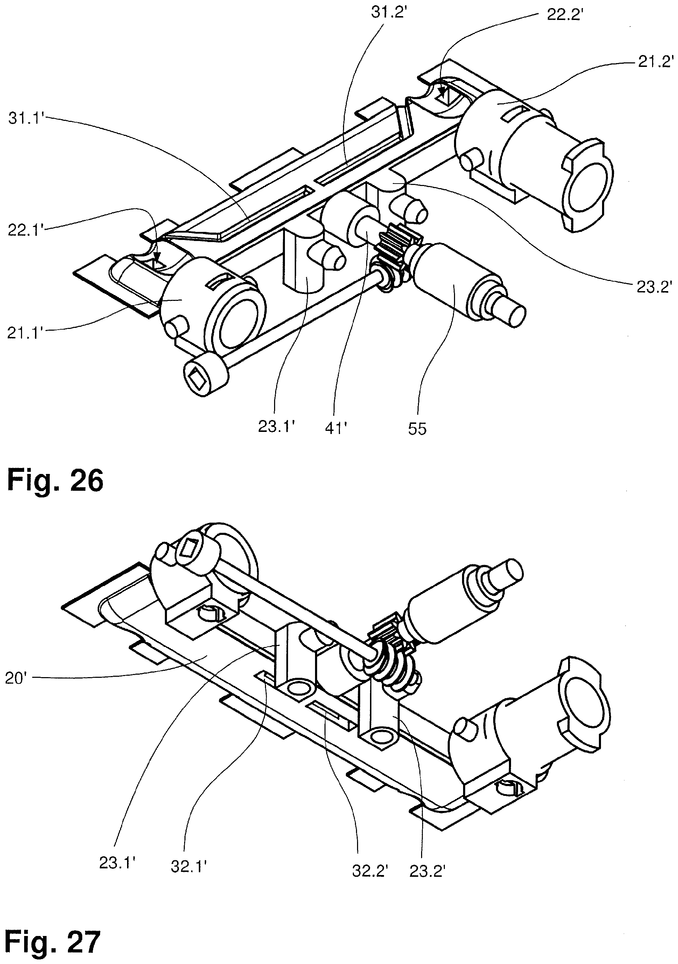

Figuren 24 und 25 zeigen Ansichten eines Behälters 15' mit mehreren Innenräumen 17.1' und 17.2' einer weiteren Ausführungsform einer erfindungsgemässen Vorrichtung. Gehäuseteile und Injektionsvorrichtung der Vorrichtung sind nicht dargestellt und können mit geringfügigen Abwandlungen gemäss den vorherigen Abbildungen ausgebildet sein. Figuren 26 und 27 zeigen äussere Schrägansichten des Verschlussstücks 20' von oben und von unten. Figuren 24 bis 27 sind im Folgenden gemeinsam beschrieben.

Die Innenräume 17.1' und 17.2' sind fluiddicht gegeneinander abgegrenzt. Die beiden Innenräume 17.1' und 17.2' werden durch eine zusätzliche Verbindungsnaht der Folienlagen in Richtung von A bereitgestellt. Der Behälter 15' weist ein einzelnes Verschlussstück 20' auf, an welches beide Innenräume 17.1' und 17.2' anschliessen. Das Verschlussstück 20' weist zwei Füllports 21.1' und 21.2' auf, welche jeweils einem der Innenräume 17.1' und 17.2' zugeordnet sind und über Füllkanäle 22.1' und 22.2' mit diesen kommunizieren.

Am Verschlussstück 20' ist eine zentrale Taumelkolbenpumpe 40' mit einem Kolben 41' ausgebildet. Der Kolben 41' ragt von aussen in einen Hubraum des Verschlussstücks 20' hinein. An einem nach aussen ragenden Abschnitt des Kolbens 41' ist ein Linearantrieb 55 gekoppelt, welcher als Hubantrieb eine Hubbewegung des Kolbens 41' antreibt. Eine Drehbewegung des Kolbens 41' wird analog der Vorrichtung 1 über ein Antriebsgetriebe umfassend ein Schneckenrad 51' erreicht, welches in ein Zahnrad 43' des Kolbens 41' eingreift. Das Schneckenrad 51' ist über ein Kopplungsmittel 52' an einen Drehantrieb (nicht dargestellt) ankoppelbar. Aufgrund des separaten Hubantriebs 55 entfällt die Steuerfläche 29 sowie der Läufer 44 des Kolbens, wie sie z.B. die Vorrichtung 1 aufweist.

Am Verschlussstück 20' sind zwei Abgabeports 23.1' und 23.2' ausgebildet, welche weitgehend analog dem Abgabeport 23 der Vorrichtung 1 ausgebildet sind. Die Abgabeports 23.1' und 23.2' sind jeweils mit Abgabekanälen 32.1' und 32.2' in Fluidkommunikation. Die Abgabekanäle 32.1' und 32.2' münden an jeweils einer Abführungsöffnung (nicht dargestellt) in den Hubraum.

Weiter sind zwei Entnahmekanäle 31.1' und 31.2' am Verschlussstück 20' ausgebildet, welche in jeweils einen der Innenräume 17.1' und 17.2' münden. Die Entnahmekanäle 31.1' und 31.2' münden zudem an jeweils einer Zuführungsöffnung (nicht dargestellt) in den Hubraum. Die Zuführungsöffnungen und die Abführungsöffnungen sind dabei in gleichem axialem Abstand mantelseitig an der Innenwand des Hubraums angeordnet. Der Hubraum kann somit mit einer Längsrille des Kolbens 41' je nach Drehstellung selektiv mit jeweils einer der Öffnungen in Fluidkommunikation gebracht werden. Die zentrale Taumelkolbenpumpe 40' erlaubt somit eine selektive Entnahme von Fluiden aus den Innenräumen 17.1' und 17.2' sowie eine selektive Beschickung der Abgabeports 23.1' und 23.2'.

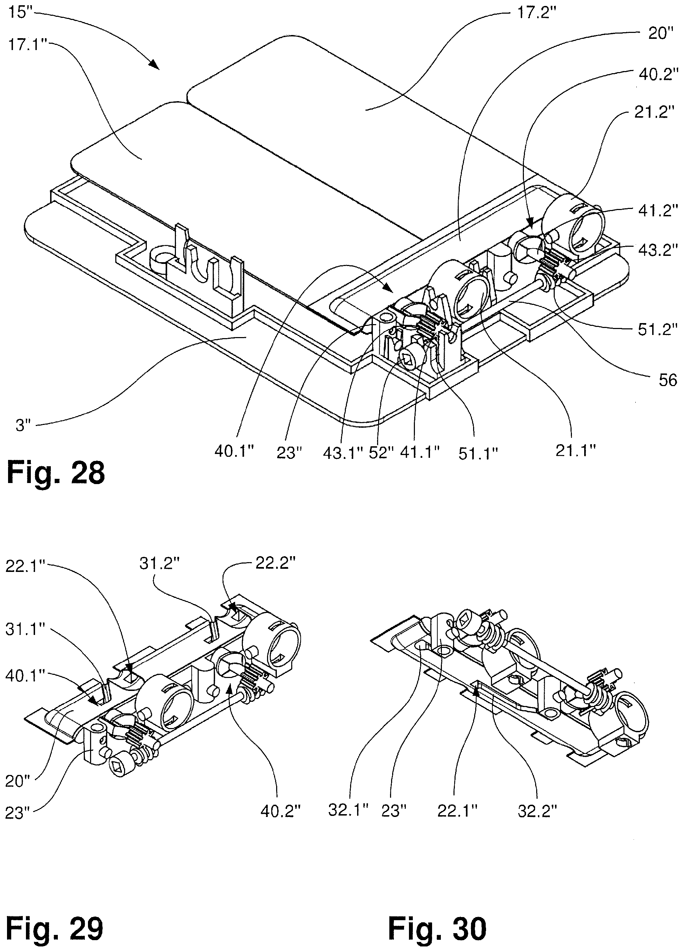

Figur 28 zeigt eine Schrägansicht eines weiteren Behälters 15" mit mehreren Innenräumen 17.1" und 17.2" einer weiteren Ausführungsform einer erfindungsgemässen Vorrichtung, welcher auf einer Grundplatte 3" eines Gehäuses der Vorrichtung angeordnet ist. Der Behälter 15" weist ein einzelnes Verschlussstück 20" auf, an welches die beiden Innenräume 17.1" und 17.2" anschliessen. Das Verschlussstück 20" weist zwei Füllports 21.1" und 21.2" auf, welche jeweils einem der Innenräume 17.1" und 17.2" zugeordnet sind und über Füllkanäle 22.1" und 22.2" mit diesen kommunizieren. Figuren 29 und 30 zeigen Schrägansichten des Verschlussstücks 20" von oben und von unten. Die Fig. 28 bis 30 sind im Folgenden gemeinsam beschrieben.

Am Verschlussstück 20" seid zwei Taumelkolbenpumpen 40.1" und 40.2" mit jeweils einem Kolben 41.1" und 41.2" ausgebildet. Die Kolben 41.1" und 41.2" ragen jeweils von aussen in einen Hubraum (nicht sichtbar) des Verschlussstücks 20'' hinein. An einem nach aussen ragenden Abschnitt der Kolben 41.1" und 41.2" ist über eine Kopplungsstange 56 ein einzelner Drehantrieb ankoppelbar. Die Kopplungsstange 56 weist zwei Schneckenräder 51.1" und 51.2" auf, welche über jeweils ein Zahnrad 43.1" und 43.2" an die Kolben 41.1" und 41.2" gekoppelt sind. An der Kopplungsstange 56 ist ein Kopplungsmittel 52" ausgebildet, über welches ein Drehantrieb ankoppelbar ist. Auf diese Weise können beide Taumelkolbenpumpen 40.1" und 40.2" mit nur einem Drehantrieb angetrieben werden. Die Hubbewegung wird analog der Vorrichtung 1 über Steuerflächen des Verschlussstücks 20'' erzeugt.

Die Hubräume beider Taumelkolbenpumpen 40.1" und 40.2" kommunizieren jeweils über einen Entnahmekanal 31.1" und 31.2" mit einem der Innenräume 17.1" respektive 17.2". Ein an den Hubraum der Taumelkolbenpumpe 40.2" anschliessender Abgabekanal 32.2" mündet in den Innenraum 17.1". Ein Abgabekanal 32.1" kommuniziert mit einem Abgabeport 23", welcher weitgehend analog dem Abgabeport 23 der Vorrichtung 1 ausgebildet ist.

Die Taumelkolbenpumpe 40.2" fördert somit ein Fluid aus dem Innenraum 17.2" in den Innenraum 17.1", während die Taumelkolbenpumpe 40.1", aufgrund der Kopplung über die Kolbenstange 56, gleichzeitig ein Fluid aus dem Innenraum 17.1" zum Abgabeport 23" fördert. Auf diese Weise können z.B. zwei getrennt gelagerte Fluide vor der Abgabe gemischt werden oder es kann im Innenraum 17.1" ein festes Medikament vorhanden sein, welches im Fluid des zweiten Innenraums 17.2" vor der Abgabe gelöst werden kann. Selbstverständliche kann die Hubbewegung der beiden Pumpen auch in diesem Fall entkoppelt sein und z.B. über separate Hubantriebe, analog der Ausführungsform der Fig. 24 bis 27, erzeugt werden.

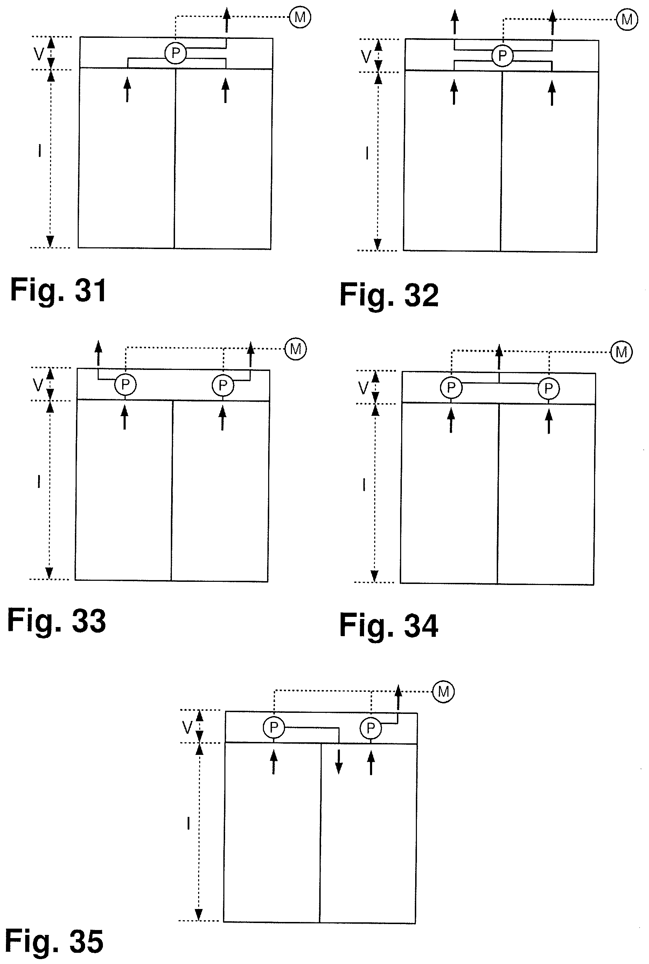

Figuren 31 bis 35 zeigen verschiedene Funktionsschemata, wie die Innenräume eines Doppelkammerbehälters mit ein oder zwei Pumpen P fluidmässig geschaltet sein können. M bezeichnet einen Pumpenantrieb, V bezeichnet dabei die Bereiche des Verschlussstücks und I die Bereiche der Innenräume. Das Schema der Fig. 32 entspricht dabei der Ausführungsform der Fig. 24 bis 27 und das Schema der Fig. 35 der Ausführungsform der Fig. 28 bis 30. Es erschliesst sich unmittelbar, wie die entsprechenden Anordnungen auch auf Behälter mit mehr als zwei Innenräumen erweiterbar sind.

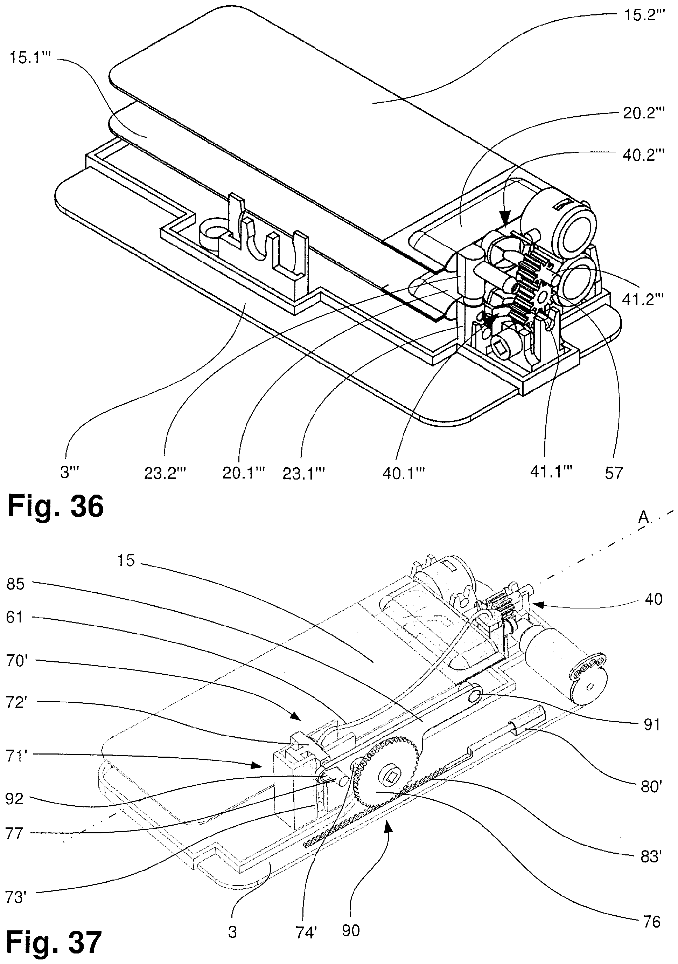

Figur 36 zeigt schliesslich eine weitere Anordnung für eine erfindungsgemässe Vorrichtung, bei welcher mehrere separate Behälter 15.1" und 15.2''' zur Anwendung kommen. Die Behälter 15.1''' und 15.2''' weisen jeweils ein Verschlussstück 20.1''' und 20.2''' auf, welche analog dem Verschlussstück 20 der Vorrichtung 1 ausgebildet sind. Die Behälter 15.1''' und 15.2''' sind übereinander auf einer Grundplatte 3''' gestapelt angeordnet. Dabei sind Abgabeports 23.1''' und 23.2''' fluidmässig aneinandergekoppelt. Jedem Behälter ist eine Taumelkolbenpumpe 40.1''' und 40.2''' zugeordnet. Kolben 41.1''' und 41.2''' der Taumelkolbenpumpen 40.1''' und 40.2''' sind über ein Kopplungszahnrad 57 miteinander gekoppelt. Auf diese Weise braucht nur einer der Kolben 41.1''' von einem Drehantrieb angetrieben zu werden.

Figur 37 zeigt eine weitere Ausführungsform einer erfindungsgemässen Vorrichtung in einer schrägen Teilansicht ohne Gehäuse. Behälter 15 sowie Taumelkolbenpumpe 40 sind analog der Ausführungsform 1 z.B. der Fig. 1 bis 9 ausgebildet. Im Gegensatz dazu ist eine Setzvorrichtung 70' sowie ein Setzantrieb 80' wie im Folgenden beschrieben ausgebildet. Die Setzvorrichtung 70' befindet sich in einer Bereitschaftsposition.

Die Setzvorrichtung 70' weist eine Führungsvorrichtung 71' mit einen Läufer 72' auf, der senkrecht zur Grundplatte 3 verschiebbar an Führungsschienen 73' geführt gelagert ist. Der Läufer 72' ist fest an der als Hohlnadel 61 ausgebildeten Einstechkanüle bei deren distalen Endbereich 65 angeordnet. Die Hohlnadel 61 sowie die daran gestützte Verweilkanüle 67 sind im Wesentlichen analog der Vorrichtung 1 ausgebildet.

Der Läufer 72' weist einen fest am Läufer 72' angeordneten Nocken 77 auf, welcher in einen Bereich des Antriebsmoduls 6 ragt. Im Antriebsmodul 6 (Gehäuse nicht dargestellt) ist ein Setzgetriebe 90 sowie der Setzantrieb 80' ausgebildet. Der Setzantrieb 80' ist als Linearantrieb ausgebildet und weist wenigstens eine elektromagnetische Spule (Solenoid) auf. In den Solenoid ragt eine Zahnstange 83', welche mit dem Solenoid derart zusammenwirkt, dass bei dessen Aktivierung des Solenoids eine Längsverschiebung der Zahnstange 83' auslösbar ist. Die Zahnstange 83' ist dabei weitgehend parallel zu A in der Antriebseinheit 6 angeordnet.

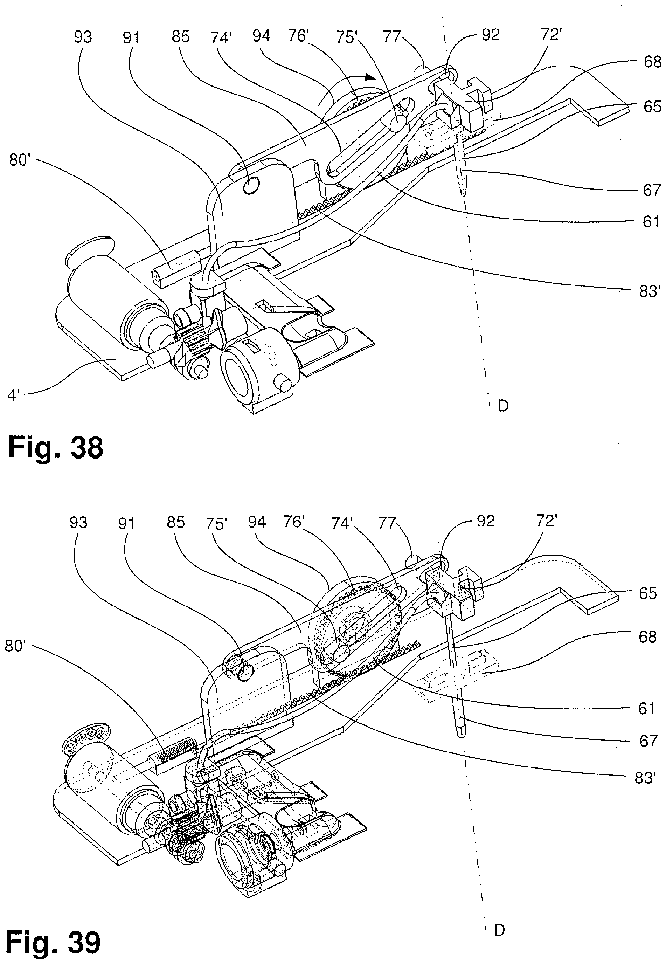

Die Zahnstange 83' steht dabei mit einem umfangseitigen Zahnkranz einer Rotationsscheibe 76' des Setzgetriebes 90 im Eingriff. Die Rotationsscheibe 76' ist an einem Lagerbock 94 (siehe z.B. Fig. 38) der Grundplatte 4' des Antriebsmoduls 6 um eine Drehachse senkrecht zu A und parallel zur Grundplatte 3 rotierbar gelagert. Bei einer Verschiebung der Zahnstange 83' kann somit eine Rotation der Rotationsscheibe 76' um deren Drehachse bewirkt werden. Die Rotationsscheibe 76' weist einen fest daran angebrachten und zur Setzvorrichtung 70' hin gerichteten Nocken 75' auf.

An einem Lagerbock 93 der Grundplatte 4' des Antriebsmoduls 6 (siehe z.B. Fig. 38) ist ein Hebelarm 85 des Setzgetriebes 90 an einem Schwenklager 91 schwenkbar gelagert. Der Hebelarm 85 erstreckt sich weitgehend parallel zu A von einem vorderen Bereich der Vorrichtung, d.h. einem in Längsrichtung A bei der Taumelkolbenpumpe 40 angeordneten Bereich, nach hinten zur Führungsvorrichtung 71' der Setzvorrichtung 70. Am Hebelarm 85 ist eine als Langloch ausgebildete Längsführung 74' ausgebildet, welche zur Einstechrichtung D leicht geneigt ausgerichtet ist und in welche der Nocken 75' der Rotationsscheibe 76' eingreift. Der Nocken 77 des Läufers 72' wiederum greift in eine weitere als Langloch ausgebildeten Längsführung 92 des Hebelarms 85 ein. Die Längsführung 92 ist dabei in einem Endbereich an einem freien Längsende des Hebelarms 85 angeordnet. Der Nocken 77 bildet ein Kopplungsmittel, über welche das Setzgetriebe 90 an die Führungsvorrichtung 71' ankoppelbar ist.

Figur 38 zeigt die Vorrichtung der Fig. 37 in einer weiteren Teilansicht von der Seite des Behälters 15 her auf die Setzvorrichtung 70'. Der Behälter 15 ist nicht dargestellt, um den Blick auf die Setzvorrichtung 70' freizugeben. Ebenso sind zur besseren Übersichtlichkeit die Grundplatte 3 und weitere Elemente der Vorrichtung nicht dargestellt. Die Setzvorrichtung 70' befindet sich in einer Bereitschaftsposition. Figur 39 zeigt eine analoge Ansicht wie Fig. 38, wobei sich die Setzvorrichtung 70' in der Endposition befindet. Figuren 38 und 39 sind im Folgenden gemeinsam beschrieben.

Zum Übergang von der Bereitschaftsposition in die Endposition wird die Rotationsscheibe 76' über die Zahnstange 83' in Rotation versetzt (siehe Pfeil). Der Nocken 75' gleitet dabei in dem Langloch 74' und übt gleichzeitig eine Kraft auf den Hebelarm 85 zur Grundplatte 3 hin aus. Dabei wird der Hebelarm 85 zur Grundplatte 3 hin verschwenkt. Über das Langloch 92 nimmt der Hebelarm 85 den Nocken 77 des Läufers 72' mit und verschiebt den Läufer 72' längs den Führungsschienen 73' zur Grundplatte 3 hin. Dabei wird die Hohlnadel 61 mit ihrem distalen Endbereich 65 und der daran gestützten Verweilkanüle 67 durch die Setzöffnung 11 (nicht dargestellt) in die Setzposition (nicht dargestellt) ausgefahren. In der Setzposition verrastet der Rastkragen 68 der Verweilkanüle 67 wie im Zusammenhang mit Fig. 10 und 11 beschrieben am Rastkranz 9 (nicht dargestellt).

Durch weitere Rotation der Rotationsscheibe 76' in dieselbe Richtung kehrt der Nocken 75' seine Bewegungsrichtung parallel zur Einstechrichtung D um, wodurch der Hebelarm 85 wieder von der Grundplatte 3 weg verschwenkt wird. Über den Nocken 77 wird der Läufer 72' somit von der Grundplatte 3 weg in den Führungsschienen 73' verschoben. Dabei wird der distale Endbereich 65 zurückgezogen, wobei die Verweilkanüle 67 in der gesetzten Position verbleibt. Die Setzvorrichtung 70' ist somit in der Endposition (siehe Fig. 39). Die Neigung der Längsführung 74' bezüglich der Einstechrichtung D ermöglicht, dass für das Einfahren eine geringere Rotation der Rotationsscheibe 76' erforderlich ist, als für das Ausfahren. Die Rotationsscheibe 76' braucht somit sowohl zum Ausfahren als auch zum Einfahren der Hohlnadel 61 nur in eine Richtung rotiert zu werden. Auf diese Weise kann der Linearantrieb 80' zur Bewegung der Zahnstange 83' in nur einer Bewegungsrichtung ausgebildet sein.

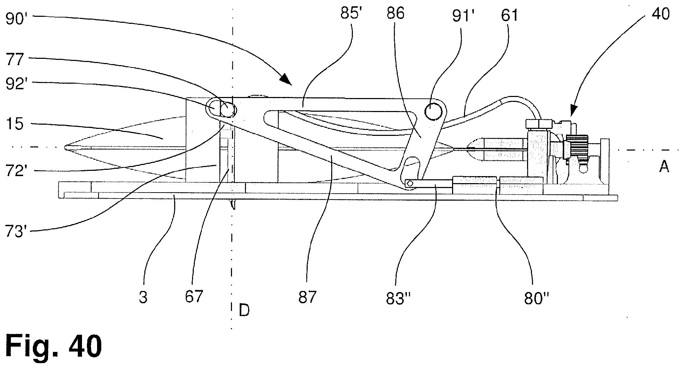

Figur 40 zeigt eine weitere Ausführungsform einer erfindungsgemässen Vorrichtung in einer seitlichen Teilansicht ohne Gehäuse. Der Behälter 15 sowie die Taumelkolbenpumpe 40 sind analog der Ausführungsform 1 z.B. der Fig. 1 bis 9 ausgebildet. Die Vorrichtung der Fig. 40 weist dieselbe Führungsvorrichtung 71' wie die Vorrichtung der Fig. 37 bis 39 auf, insbesondere umfassend den Läufer 72', die Führungsschienen 73' sowie den Nocken 77. Im Gegensatz dazu ist ein Setzgetriebe 90' und ein Setzantrieb 80" wie im Folgenden beschrieben ausgebildet.

Der Setzantrieb 80" weist zwei Solenoide auf, mit welchen eine parallel zur Längsrichtung A angeordnete Schubstange 83" geradlinig in beide Richtungen bewegt werden kann. Das Setzgetriebe 90' umfasst einen Hebelarm 85', welcher weitgehend analog dem Hebelarm 85 an einem Lagerbock (nicht dargestellt) der Grundplatte 4' des Antriebsmoduls 6 an einem Schwenklager 91' schwenkbar gelagert ist. Der Hebelarm 85' weist ebenfalls analog dem Hebelarm 85 ein Langloch 92' auf, in welches der fest mit dem Läufer 72' der Führungsvorrichtung 71' verbundene Nocken 77 eingreift. Bei einem Verschwenken des Hebelarms 85' zur Grundplatte 3 hin, wird somit analog der Vorrichtung der Fig. 37 bis 39 der Läufer 72' zur Grundplatte 3 hin verschoben und bei einem Verschwenken weg von der Grundplatte 3 von dieser weg. Wie im Zusammenhang mit Fig. 37 und 39 beschrieben, erfolgt somit ein Ausfahren und Einfahren des distalen Endbereichs 65 der Hohlnadel 61 durch die Setzöffnung 11 (nicht dargestellt) zum Setzen der Verweilkanüle 67.

Der Hebelarm 85' weist nach Art eines Kniehebels einen Armfortsatz 86 auf, welcher sich ausgehend vom Schwenklager 91' zur Grundplatte 3 hin erstreckt. Hebelarm 85' und Armfortsatz 86 sind starr miteinander verbunden und schliessen einen Winkel derart ein, dass bei einer Kraftwirkung auf ein freies Längsende des Armfortsatzes 86 der Hebelarm 85' um das Schwenklager 91' verschwenkbar ist. Der eingeschlossen Winkel kann dabei bevorzugt etwa 60 bis 120 Grad betragen, um eine vorteilhafte Kraftübertragung vom Armfortsatz 86 auf den Hebelarm 85' zu erreichen. Die freien Längsenden des Hebelarms 85' und des Armfortsatzes 86 sind zur besseren mechanischen Stabilität mit einem Versteifungsbalken 87 miteinander verbunden.

Die Schubstange 83" des Setzantriebs 80" greift im Bereich des freien Längsendes des Armfortsatzes 86 an, wobei der Angriffspunkt bezüglich des Armfortsatzes 86 verschiebbar ist. Auf diese Weise wird erreicht, dass eine relative Verschiebung aufgrund der Linearbewegung der Schubstange 83" und der Schwenkbewegung des Armfortsatzes 86 ausgeglichen werden kann.

Es erschliesst sich unmittelbar, dass bei einer Zugbewegung der Schubstange 83" der Hebelarm 85' über den Armfortsatz 86 zur Grundplatte 3 hin abgesenkt und bei einer Schubbewegung in ent-gegengesetzter Richtung von der Grundplatte 3 weg angehoben werden kann. Die Vorrichtung gemäss Fig. 40 zeigt somit eine Setzvorrichtung 70", bei welcher keine Rotationsscheibe zum Einsatz kommt, sondern eine geradlinige Bewegung über einen Kniehebel (Hebelarm 85', Armfortsatz 86) direkt in eine Bewegung in Einstechrichtung D umgesetzt wird.

werden kann. Die Vorrichtung gemäss Fig. 40 zeigt somit eine Setzvorrichtung 70", bei welcher keine Rotationsscheibe zum Einsatz kommt, sondern eine geradlinige Bewegung über einen Kniehebel (Hebelarm 85', Armfortsatz 86) direkt in eine Bewegung in Einstechrichtung D umgesetzt wird.

## Patentansprüche

1. Vorrichtung (1) zur Abgabe eines Fluids, an einen Patienten, umfassend einen Behälter (15) mit einem Innenraum (17) zur Aufnahme des Fluids, wobei der Behälter (15) ein im Behälter (15) angeordnetes Verschlussstück (20) umfasst, an welchem eine Abgabeöffnung (24) ausgebildet ist, über welche das Fluid aus dem Innenraum (17) abgebbar ist, wobei die Vorrichtung (1) eine von einem Förderantrieb (50) angetriebene Fördervorrichtung (40) zur Förderung des Fluids aus dem Innenraum (17) des Behälters (15) umfasst, **dadurch gekennzeichnet, dass** die Fördervorrichtung (40) fluidmässig zwischen dem Innenraum (17) und der Abgabeöffnung (24) angeordnet ist, sodass das Fluid mittels der Fördervorrichtung (40) aus dem Innenraum (17) zur Abgabeöffnung (24) förderbar ist, wobei die Fördervorrichtung (40) als ventillose Verdrängerpumpe ausgebildet ist.

2. Vorrichtung nach Anspruch 1, **dadurch gekennzeichnet, dass** der Behälter (15) zumindest teilweise kollabierbar ist.

3. Vorrichtung (1) gemäss Anspruch 1 oder 2, **dadurch gekennzeichnet, dass** die ventillose Verdrängerpumpe als Taumelkolbenpumpe (40) mit einem Kolben (41) und einem Hubraum (42) ausgebildet ist, wobei der Kolben (41) bei jeder Hubbewegung im Hubraum (42) um eine in der Hubrichtung verlaufende Achse eine Drehbewegung ausführt.

4. Vorrichtung (1) gemäss Anspruch 3, **dadurch gekennzeichnet, dass** der Hubraum (42) in Abhängigkeit einer Drehstellung des Kolbens (41), insbesondere gegebenenfalls über den Entnahmekanal (31), mit dem Innenraum (17) oder, insbesondere gegebenenfalls über den Abgabekanal (32), mit der Abgabeöffnung (24) in Fluidkommunikation bringbar ist.

5. Vorrichtung (1) gemäss einem der Ansprüche 3 oder 4, **dadurch gekennzeichnet, dass** der Hubraum (42) im Verschlussstück (20) ausgebildet ist.

6. Vorrichtung gemäss einem der Ansprüche 1 bis 5, **dadurch gekennzeichnet, dass** der Behälter (15') wenigstens einen weiteren Innenraum (17.2') umfasst, welcher gegenüber dem ersten Innenraum (17.1') derart abgetrennt ist, dass wenigstens ein weiteres Fluid getrennt vom ersten Fluid im Behälter (15') aufnehmbar ist.

7. Vorrichtung gemäss Anspruch 6, **dadurch gekennzeichnet, dass** die Fördervorrichtung (40') derart fluidmässig zwischen dem wenigstens einen weiteren Innenraum (17.2') und
- der Abgabeöffnung oder
- wenigstens einer weiteren Abgabeöffnung oder
- einem anderen der Innenräume (17.1')
angeordnet ist, dass das wenigstens eine weitere Fluid mittels der Fördervorrichtung (40') aus dem wenigstens einen weiteren Innenraum (17.2') zur Abgabeöffnung oder zur wenigstens einen weiteren Abgabeöffnung oder in den anderen der Innenräume (17.1') förderbar ist.

8. Vorrichtung gemäss Anspruch 6, **dadurch gekennzeichnet, dass** wenigstens eine weitere Fördervorrichtung (40.2") in der Vorrichtung vorhanden ist, welche dem wenigstens einen weiteren Innenraum (17.2'') zugeordnet ist und derart fluidmässig zwischen dem wenigstens einen weiteren Innenraum (17.2") und
- der Abgabeöffnung oder
- wenigstens einer weiteren Abgabeöffnung oder
- einem anderen der Innenräume (17.1'')
angeordnet ist, dass das wenigstens eine weitere Fluid mittels der wenigstens einen weiteren Fördervorrichtung (40.2'') aus dem wenigstens einen weiteren Innenraum (17.2'') zur Abgabeöffnung oder zur wenigstens einen weiteren Abgabeöffnung oder in den anderen der Innenräume (17.1'') förderbar ist.

9. Vorrichtung nach Anspruch 8, **dadurch gekennzeichnet, dass** die wenigstens eine weitere Fördervorrichtung (40.2") der ersten Fördervorrichtung (40.1") weitgehend gleichartig ist.

10. Vorrichtung gemäss einem der Ansprüche 1 bis 9, **dadurch gekennzeichnet, dass** mehrere Behälter (15.1''', 15.2''') vorhanden sind, und jedem Behälter (15.1''', 15.2''') wenigstens eine Fördervorrichtung (40.1''', 40.2''') zugeordnet ist.

11. Vorrichtung gemäss Anspruch 3 und einem der Ansprüche 8 bis 10, **dadurch gekennzeichnet, dass** die Drehbewegungen der Kolben der Fördervorrichtungen, bevorzugt mechanisch, gekoppelt sind.

12. Vorrichtung (1) gemäss einem der Ansprüche 1 bis 11, wobei die Vorrichtung eine Injektionsvorrichtung (60) zur kontinuierlichen subkutanen Abgabe des Fluids oder gegebenenfalls der Fluide an den Patienten umfasst.

13. Vorrichtung (1) nach Anspruch 12 zur Abgabe eines Fluids, an einen Patienten, umfassend einen Behälter (15) zur Aufnahme des Fluids, mit einem Gehäuse (2), welches aussenseitig eine Kontaktfläche (10) aufweist, mit welcher die Vorrichtung (1) an einen Körper des Patienten anlegbar ist, wobei eine Injektionsvorrichtung (60) mit einer von einem distalen Endbereich (65) einer Einstechkanüle (61) gestützten flexiblen transkutanen Verweilkanüle (67) vorhanden ist, wobei die Einstechkanüle (61) in einer Bereitschaftsposition im Wesentlichen innerhalb des Gehäuses (2) angeordnet ist und zum Setzen der Verweilkanüle (67) mit dem distalen Endbereich (65) durch eine Setzöffnung (11) an der Kontaktfläche (10) aus dem Gehäuse (2) in eine Setzposition ausfahrbar und wieder in eine Endposition einfahrbar ist, wobei ein proximaler Endbereich (63) der Einstechkanüle (61) fluiddicht an eine Abgabeöffnung (24) angeschlossen ist, an welcher das Fluid aus dem Behälter (15) abgebbar ist, wobei eine Führungsvorrichtung (71) vorhanden ist, an welcher der distale Endbereich (65) der Einstechkanüle (61) beim Einfahren und Ausfahren durch die Setzöffnung (11), vorzugsweise verschiebbar, geführt ist, **dadurch gekennzeichnet, dass** die Vorrichtung (1) ein Antriebsmodul (6) und ein Abgabemodul (7) umfasst, welche durch einen Benutzer verbindbar und voneinander trennbar ausgebildet sind, wobei das Antriebsmodul (6) zumindest Teile des Förderantriebs (50), insbesondere gegebenenfalls den Drehantrieb (50), und/oder gegebenenfalls den Setzantrieb (80) der Injektionsvorrichtung (60) umfasst, und das Abgabemodul (7) zumindest den Behälter (15) sowie die Fördervorrichtung (40) und gegebenenfalls die Injektionsvorrichtung (60) aufweist.

14. Vorrichtung (1) gemäss Anspruch 13, **dadurch gekennzeichnet, dass** der proximale Endbereich (63) der Einstechkanüle (61) ortsfest und starr in der Vorrichtung (1) angeordnet ist.

15. Vorrichtung (1) gemäss Anspruch 14, **dadurch gekennzeichnet, dass** die Einstechkanüle (61) wenigstens in der Bereitschaftsposition in einem Mittelbereich (64) zwischen dem proximalen (63) und dem distalen Endbereich (65) mit einem gleich bleibenden Krümmungssinn gekrümmt ist, wobei ein eingeschlossener Winkel (α) zwischen dem proximalen Endbereich (63) und einem benachbarten Abschnitt des Mittelbereichs (64) sowie ein eingeschlossener Winkel (α') zwischen dem distalen Endbereich (65) und einem benachbarten Abschnitt des Mittelbereichs (64) jeweils kleiner als 90 Grad ist.

16. Vorrichtung (1) gemäss einem der Ansprüche 13 bis 15, **dadurch gekennzeichnet, dass** die Verweilkanüle (67), insbesondere selbsttätig, am Gehäuse (2) arretierbar ist, wenn die Einstechkanüle (61) in der Setzposition ist.

17. Vorrichtung (1) gemäss einem der Ansprüche 13 bis 16, **dadurch gekennzeichnet, dass** ein Setzantrieb (80) vorhanden ist, mit welchem der distale Endbereich (65) der Einstechkanüle (61) über ein Setzgetriebe zum Setzen der Verweilkanüle (67) aus- und wieder einfahrbar ist.

18. Vorrichtung (1) gemäss Anspruch 17, **dadurch gekennzeichnet, dass** der Setzantrieb (80) einen Drehantrieb und das Setzgetriebe ein Schneckengetriebe umfasst, über welches der Drehantrieb an die Einstechkanüle (61), insbesondere gegebenenfalls an den Läufer (72) der Führungsvorrichtung (71), ankoppelbar oder angekoppelt ist.

19. Vorrichtung (1) gemäss Anspruch 17, **dadurch gekennzeichnet, dass** der Setzantrieb (80) einen Linearantrieb und das Setzgetriebe einen schwenkbar gelagerten Kniehebel umfasst, über welchen der Linearantrieb an die Einstechkanüle (61), ankoppelbar oder angekoppelt ist.

20. Vorrichtung (1) gemäss einem der Ansprüche 17 bis 19, **dadurch gekennzeichnet, dass** das Setzgetriebe eine Längsführung (74) aufweist, welche weitgehend quer zur Einstechrichtung (D) angeordnet ist und in welche ein Nocken des Setzgetriebes in der Längsführung (74) verschiebbar eingreift.

## Claims

1. Device (1) for dispensing a fluid to a patient, comprising a container (15) having an interior space (17) for receiving the fluid, wherein the container (15) comprises a closure piece (20) arranged in the container (15), on which closure piece a dispensing opening (24) is formed, via which the fluid from the interior space (17) can be dispensed, wherein the device (1) comprises a conveyor (40), driven by a conveyor drive (50), for conveying the fluid out of the interior space (17) of the container (15), **characterized in that** the conveyor (40) is arranged fluidically between the interior space (17) and the dispensing opening (24), such that the fluid can be conveyed by means of the conveyor (40) from the interior space (17) to the dispensing opening (24), wherein the conveyor (40) is configured as a valveless positive displacement pump.

2. Device according to claim 1, **characterized in that** the container (15) is at least partially collapsible.

3. Device (1) according to claim 1 or 2, **characterized in that** the valveless positive displacement pump is configured as a nutating pump (40) with a piston (41) and a displacement (42), wherein the piston (41) performs a rotational movement about an axis running in the stroke direction during each stroke movement in the displacement space (42).

4. Device (1) according to claim 3, **characterized in that** the displacement (42), as a function of a rotational position of the piston (41), can be brought into fluid communication with the interior (17), in particular optionally via the withdrawal channel (31) or, with the dispensing opening (24), in particular optionally via the dispensing channel (32).

5. Device (1) according to one of claims 3 or 4, **characterized in that** the displacement (42) is formed in the closure piece (20).

6. Device according to one of claims 1 to 5, **characterized in that** the container (15') comprises at least one further interior space (17.2') which is separated from the first interior space (17.1') in such a way that at least one further fluid can be received in the container (15') separately from the first fluid.

7. Device according to claim 6, **characterized in that** the conveyor (40') is fluidically arranged between the at least one further interior space (17.2') and
- the dispensing opening or
- at least one further dispensing opening or
- another of the interior spaces (17.1')
in such a way that the at least one further fluid can be conveyed by means of the conveyor (40') from the at least one further interior space (17.2') to the dispensing opening or to the at least one further dispensing opening or into the other of the interior spaces (17.1').

8. Device according to claim 6, **characterized in that** at least one further conveyor (40.2") is provided in the device, which is assigned to the at least one further interior space (17.2") and is fluidically arranged between the at least one further interior space (17.2") and
- the dispensing opening or
- at least one further dispensing opening or
- another of the interior spaces (17.1")
in such a way that at least one further fluid can be conveyed by means of the at least one further conveyor (40.2") from the at least one further interior space (17.2") to the dispensing opening or to the at least one further dispensing opening or into the other of the interior spaces (17.1").

9. Device according to claim 8, **characterized in that** the at least one further conveyor (40.2") is substantially similar to the first conveyor (40.1").

10. Device according to one of claims 1 to 9, **characterized in that** a plurality of containers (15.1''', 15.2"') are provided, and at least one conveyor (40.1''', 40.2''') is assigned to each container (15.1''', 15.2''').

11. Device according to claim 3 and one of claims 8 to 10, **characterized in that** the rotational movements of the pistons of the conveyors are coupled, preferably mechanically.

12. Device (1) according to one of claims 1 to 11, wherein the device comprises an injection device (60) for continuous subcutaneous dispensing of the fluid or, optionally, the fluids to the patient.

13. Device (1) according to claim 12 for dispensing a fluid to a patient, comprising a container (15) for receiving the fluid, having a housing (2), which has a contact surface (10) on the outside, with which contact surface the device (1) can be placed on the patient's body, wherein an injection device (60) is provided, having a flexible transcutaneous indwelling cannula (67) supported by a distal end region (65) of a puncture cannula (61), wherein the puncture cannula (61) is arranged, in a ready position, substantially within the housing (2) and, for placing the indwelling cannula (67) with the distal end region (65), can be extended out of the housing (2) into a placement position through a placement opening (11) on the contact surface (10) and can be retracted again into an end position, wherein a proximal end region (63) of the puncture cannula (61) is connected in a fluid-tight manner to a dispensing opening (24) to which the fluid from the container (15) can be dispensed, wherein a guide device (71) is provided, to which the distal end region (65) of the puncture cannula (61) is guided, preferably slidably, upon extension and retraction through the placement opening (11), **characterized in that** the device (1) comprises a drive module (6) and a dispensing module (7), which are configured to be connectible to and separable from one another by a user, wherein the drive module (6) comprises at least parts of the conveyor drive (50), in particular optionally the rotary drive (50), and/or optionally the placement drive (80) of the injection device (60), and the dispensing module (7) has at least the container (15) and the conveyor (40) and optionally the injection device (60).

14. Device(1) according to claim 13, **characterized in that** the proximal end region (63) of the puncture cannula (61) is arranged in a stationary and rigid manner in the device (1).

15. Device (1) according to claim 14, **characterized in that** the puncture cannula (61) is curved at least in the ready position in a central region (64) between the proximal (63) and the distal (65) end region with a constant sense of curvature, wherein an angle (**α**) enclosed between the proximal end region (63) and an adjacent portion of the central region (64) and an angle (α') enclosed between the distal end region (65) and an adjacent portion of the central region (64) is in each case less than 90 degrees.

16. Device (1) according to one of claims 13 to 15, **characterized in that** the indwelling cannula (67) can be locked, in particular automatically, on the housing (2) when the puncture cannula (61) is in the placement position.

17. Device (1) according to one of claims 13 to 16, **characterized in that** a placement drive (80) is provided, by means of which the distal end region (65) of the puncture cannula (61) can be extended and retracted via a placement gearing for placing the indwelling cannula (67).

18. Device (1) according to claim 17, **characterized in that** the placement drive (80) comprises a rotary drive and the placement gearing comprises a worm gear via which the rotary drive can be coupled or is coupled to the puncture cannula (61), in particular optionally to the runner (72) of the guide device (71).

19. Device (1) according to claim 17, **characterized in that** the placement drive (80) comprises a linear drive and the placement gearing comprises a pivotably mounted toggle lever via which the linear drive can be coupled or is coupled to the puncture cannula (61).

20. Device (1) according to one of claims 17 to 19, **characterized in that** the placement gearing has a longitudinal guide (74) which is arranged substantially transversely to the injection direction (D) and into which a cam of the placement gearing slidingly engages in the longitudinal guide (74).

## Revendications

1. Dispositif (1) de distribution de fluide à un patient, comprenant un récipient (15) avec un espace intérieur (17) pour recevoir le fluide, dans lequel le récipient (15) comprend une pièce de fermeture (20) qui est disposée dans le récipient (15) et sur laquelle est formé un orifice de distribution (24), par lequel le fluide peut être distribué à partir de l'espace intérieur (17), le dispositif (1) comprenant un dispositif de transport (40) entraîné par un entraînement de transport (50) pour transporter le fluide depuis l'espace intérieur (17) du récipient (15), **caractérisé en ce que** le dispositif de transport (40) est disposé fluidiquement entre l'espace intérieur (17) et l'orifice de distribution (24), de sorte que le fluide peut être transporté au moyen du dispositif de transport (40) de l'espace intérieur (17) à l'orifice de distribution (24), le dispositif de transport (40) étant conçu comme une pompe volumétrique sans clapet.

2. Dispositif selon la revendication 1, **caractérisé en ce que** le récipient (15) est au moins partiellement aplatissable.

3. Dispositif (1) selon la revendication 1 ou 2, **caractérisé en ce que** la pompe volumétrique sans clapet est conçue comme une pompe à piston oscillant (40) avec un piston (41) et une cylindrée (42), dans laquelle le piston (41) effectue un mouvement de rotation dans la cylindrée (42) sur un axe s'étendant dans la direction de la course pendant chaque mouvement de course.

4. Dispositif (1) selon la revendication 3, **caractérisé en ce que** la cylindrée (42), en fonction d'une position de rotation du piston (41), peut être mise en communication fluidique avec l'espace intérieur (17), en particulier le cas échéant par le canal de prélèvement (31), en particulier le cas échéant par le canal de distribution (32), avec l'orifice de distribution (24).

5. Dispositif (1) selon l'une des revendications 3 ou 4, **caractérisé en ce que** la cylindrée (42) est formée dans la pièce de fermeture (20).

6. Dispositif selon l'une des revendications 1 à 5, **caractérisé en ce que** le récipient (15') comprend au moins un autre espace intérieur (17.2') qui est séparé du premier espace intérieur (17.1') de telle manière qu'au moins un autre fluide peut être reçu séparément du premier fluide dans le récipient (15').

7. Dispositif selon la revendication 6, **caractérisé en ce que** le dispositif de transport (40') est disposé fluidiquement entre l'au moins un autre espace intérieur (17.2') et
- l'orifice de distribution ou
- au moins un autre orifice de distribution ou
- un autre des espaces intérieurs (17.1'),
**en ce que** l'au moins un autre fluide peut être transporté au moyen du dispositif de transport (40') depuis l'au moins un autre espace intérieur (17.2') vers l'orifice de distribution ou vers l'au moins un autre orifice de distribution ou dans l'autre des espaces intérieurs (17.1').

8. Dispositif selon la revendication 6, **caractérisé en ce qu'**au moins un autre dispositif de transport (40.2") est présent dans le dispositif, qui est associé à l'au moins un autre espace intérieur (17.2") et qui est disposé entre l'au moins un autre espace intérieur (17.2") et
- l'orifice de distribution ou
- au moins un autre orifice de distribution ou
- un autre des espaces intérieurs (17.1"),
**en ce que** l'au moins un autre fluide peut être transporté au moyen de l'au moins un autre dispositif de transport (40.2") depuis l'au moins un autre espace intérieur (17.2") vers l'orifice de distribution ou vers l'au moins un autre orifice de distribution ou dans l'autre des espaces intérieurs (17.1").

9. Dispositif selon la revendication 8, **caractérisé en ce que** l'au moins un autre dispositifs de transport (40.2") sont essentiellement similaires au premier dispositif de transport (40.1").

10. Appareil selon l'une des revendications 1 à 9, **caractérisé en ce que** plusieurs récipients (15.1''', 15.2"') sont présents, et au moins un dispositif de transport (40.1''', 40.2''') est associé à chaque récipient (15.1''', 15.2''').

11. Dispositif selon la revendication 3 et l'une des revendications 8 à 10, **caractérisé en ce que** les mouvements de rotation des pistons des dispositifs de transport sont couplés, de préférence mécaniquement.

12. Dispositif (1) selon l'une des revendications 1 à 11, le dispositif comprenant un dispositif d'injection (60) pour la distribution sous-cutanée continue du fluide ou, éventuellement, des fluides au patient.

13. Dispositif (1) selon la revendication 12 pour la distribution de fluide à un patient, comprenant un récipient (15) pour recevoir le fluide, avec un boîtier (2) qui présente à l'extérieur une surface de contact (10) avec laquelle le dispositif (1) peut être placé contre un corps de patient, dans lequel un dispositif d'injection (60) avec une canule à demeure transcutanée flexible (67) soutenue par une région d'extrémité distale (65) d'une canule d'insertion (61) est présent, dans lequel la canule d'insertion (61) est disposée dans une position de veille sensiblement à l'intérieur du boîtier (2) et, pour la mise en place de la canule à demeure (67), peut être sortie du boîtier (2) avec la région d'extrémité distale (65) à travers un orifice de réglage (11) sur la surface de contact (10) dans une position de mise en place et peut être rétractée dans une position finale, dans lequel une région d'extrémité proximale (63) de la canule d'insertion (61) est reliée de manière étanche aux fluides à un orifice de distribution (24), par lequel le fluide peut être distribué à partir du récipient (15), un dispositif de guidage (71) étant prévu, par lequel la région d'extrémité distale (65) de la canule d'insertion (61) est guidée, de préférence de manière déplaçable, lors de l'insertion et du retrait à travers l'orifice de réglage (11), **caractérisé en ce que** le dispositif (1) comprend un module d'entraînement (6) et un module de distribution (7), qui peuvent être reliés et séparés l'un de l'autre par un utilisateur, le module d'entraînement (6) comprenant au moins des parties de l'entraînement de transport (50), en particulier le cas échéant l'entraînement rotatif (50), et/ou le cas échéant l'entraînement de réglage (80) du dispositif d'injection (60), et le module de distribution (7) comportant au moins le récipient (15) et le dispositif de transport (40) et le cas échéant le dispositif d'injection (60).

14. Dispositif (1) selon la revendication 13, **caractérisé en ce que** la région d'extrémité proximale (63) de la canule d'insertion (61) est disposée de manière fixe et rigide dans le dispositif (1).

15. Dispositif (1) selon la revendication 14, **caractérisé en ce que** la canule d'insertion (61) est incurvée avec un sens de courbure constant au moins dans la position de veille dans une région centrale (64) entre la région d'extrémité proximale (63) et la région d'extrémité distale (65), dans laquelle un angle inclus **(α)** entre la région d'extrémité proximale (63) et une partie adjacente de la région centrale (64) et un angle inclus **(α')** entre la région d'extrémité distale (65) et une partie adjacente de la région centrale (64) sont chacun inférieurs à 90 degrés.

16. Dispositif (1) selon l'une des revendications 13 à 15, **caractérisé en ce que** la canule à demeure (67) peut être verrouillée, en particulier automatiquement, sur le boîtier (2) lorsque la canule d'insertion (61) est en position de réglage.

17. Dispositif (1) selon l'une des revendications 13 à 16, **caractérisé en ce qu'**un entraînement de réglage (80) est présent, au moyen duquel la région d'extrémité distale (65) de la canule d'insertion (61) peut être sortie et rentrée à nouveau par l'intermédiaire d'un pignon de réglage pour le réglage de la canule à demeure (67).

18. Dispositif (1) selon la revendication 17, **caractérisé en ce que** l'entraînement de réglage (80) comprend un entraînement rotatif et le pignon de réglage comprend une vis sans fin, par lequel l'entraînement rotatif peut être couplé ou est couplé à la canule d'insertion (61), en particulier le cas échéant au rotor (72) du dispositif de guidage (71).

19. Dispositif (1) selon la revendication 17, **caractérisé en ce que** l'entraînement de réglage (80) comprend un entraînement linéaire et le pignon de réglage comprend un levier basculant monté de manière pivotante, par lequel l'entraînement linéaire peut être couplé ou est couplé à la canule d'insertion (61).

20. Dispositif (1) selon l'une des revendications 17 à 19, **caractérisé en ce que** l'entraînement de réglage présente un guide longitudinal (74) qui est disposé essentiellement transversalement par rapport à la direction d'insertion (D) et dans lequel une came de l'entraînement de réglage s'engage de manière déplaçable dans le guide longitudinal (74).
